# NEW EUROPEAN PATENT SPECIFICATION

(11) **EP 0 472 858 B2**
(45) Date of publication and mention of the opposition decision: **15.03.2000**
(45) Mention of the grant of the patent: 20.12.1995
(21) Application number: 91111026.0
(22) Date of filing: 03.07.1991
(51) Int. Cl.: A61K 7/06

(54) **Hair treatment composition**
Haarbehandlungszusammensetzung
Composition pour traiter les cheveux

(30) Priority: 04.07.1990 JP 17683990; 04.07.1990 JP 17684090; 04.07.1990 JP 17684190; 03.09.1990 JP 23299690; 03.09.1990 JP 23299790; 03.09.1990 JP 23299890; 03.09.1990 JP 23299990
(43) Date of publication of application: 04.03.1992
(73) Proprietor: KAO CORPORATION, Chuo-ku Tokyo (JP)
(72) Inventor: Kawase, Jiro, Kao Corporation, Sumida-ku, Tokyo (JP); Ogura, Yoshiko, Kao Corporation, Sumida-ku, Tokyo (JP); Yoshino, Koji, Kao Corporation, Ichikai-machi, Haga-gun, Tochigi (JP); Kawamata, Akira, Kao Corporation, Sumida-ku, Tokyo (JP)
(74) Representative: Hansen, Bernd, Dr. Dipl.-Chem.

(56) References cited:
- EP-A- 0 095 238
- EP-A- 0 287 773
- EP-A- 0 313 867
- DE-A- 3 740 926
- DE-C- 3 323 881
- GB-A- 1 553 310

## Description

### FIELD OF THE INVENTION

This invention relates to a hair treatment composition which imparts to the hair an effect of preventing hair from causing a split hair and an effect of improving hair gloss which can sustain for a long period of time.

### BACKGROUND OF THE INVENTION

As hair treatment compositions for preventing hair from causing a split hair, those containing polymer silicones as disclosed, for example, in JP-A-1-273513 (the term "JP-A" as used herein means an "unexamined published Japanese patent application") have been known. These compositions aim at preventing the hair from causing a split hair/broken hair in brushing step by improving the slippage of the surface of the hair.

In each of the aforesaid known hair treatment compositions, however, conditioning components, which are attached to the surface of the hair merely through physical adsorption, are easily removed by shampooing following the application of the hair treatment composition to the hair. Thus it cannot exert a sustained effect of preventing hair from causing a split hair or improving hair gloss.

Accordingly, it is an object of the present invention to provide a hair treatment composition which imparts to the hair an effect of preventing hair from causing a split hair and an effect of improving hair gloss which can sustain for a long period of time.

### SUMMARY OF THE INVENTION

The present inventors have conducted extensive studies in order to achieve the aforesaid object. As a result, they have found that an effect of preventing hair from causing a split hair and an effect of improving hair gloss can be achieved by treating the hair with a composition comprising a specific silicone compound together with a specific penetration accelerator, which has been adjusted to a specific pH value, or using a combination of three components involving a specific silicone compound, a specific penetration accelerator and an oxidizing agent.

As the results of subsequent studies, the present inventors have further found that a cyclic Bunte salt-modified silicone represented by the following general formula (V) is a compound which can achieve the aforesaid object and that an effect of preventing hair from causing a split hair and an effect of improving hair gloss sustaining for a long period of time can be obtained by applying the cyclic Bunte salt-modified silicone which has been adjusted to a specific pH value or combined with an oxidizing agent or a reducing agent.

Accordingly, the present invention, which has been completed based on these findings, provides a hair treatment composition which comprises:
(a) one or more compounds represented by the following general formula (I) or (II); and
(b) one or more compounds represented by the following general formula (III) or (IV);
   and the pH value thereof is adjusted to 2 to 4 or 8 to 11 at the use: 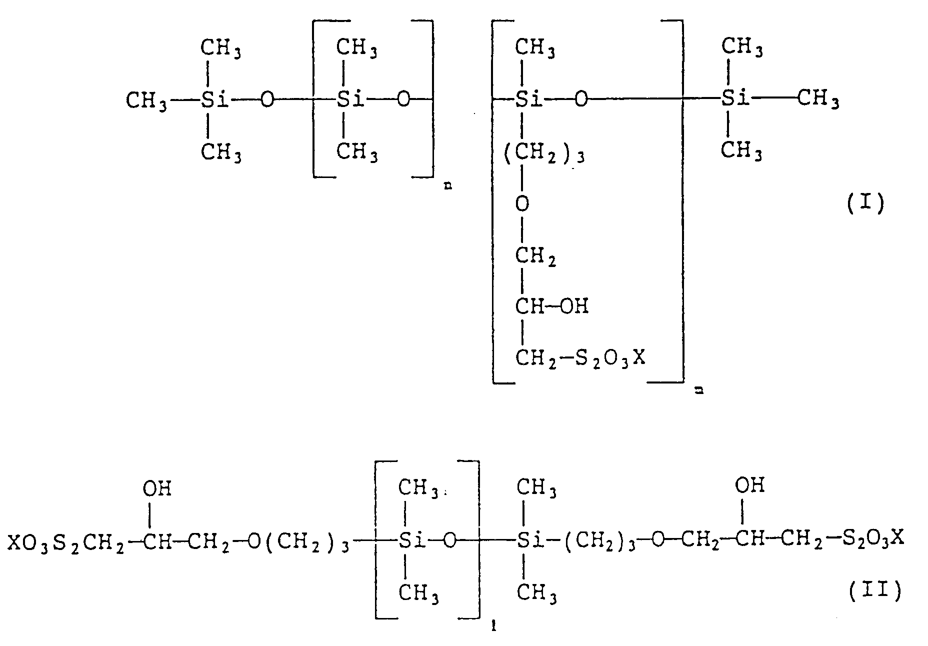 wherein X represents an alkali metal, an alkaline earth metal, an ammonium, a primary ammonium, a secondary ammonium, a tertiary ammonium or a quaternary ammonium; n is an integer of from 1 to 20; m is an integer of from 1 to 20, provided that n/m >1; and ℓ is an integer of from 1 to 20; 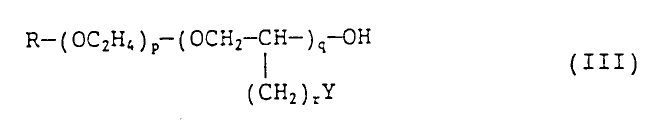 wherein R represents a hydrogen atom or an 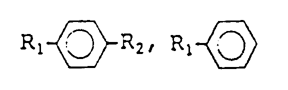 or a straight-chain or branched alkyl group having 1 to 4 carbon atoms, in which R₁ represents a hydrogen atom, a methyl group or a methoxy group, and R₂ represents a -CH₂-, -C(CH₃)₂-, -CH₂-CH-(CH₃)- or -CH = CH-CH₂- group; Y represents a hydrogen atom or a hydroxyl group; and p, q and r are each an integer of from 0 to 5, provided that at least one of p and q is 0j and provided that in the compound of formula (III) p and q may not be 0 at the same time, when R is hydrogen; 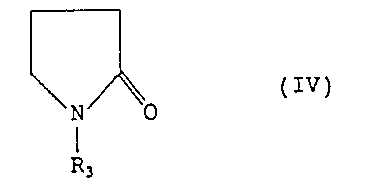 wherein R₃ represents a straight-chain or branched alkyl group having 8 to 18 carbon atoms.
   Further, the present invention provides a hair treatment composition which comprises the aforesaid components (a) and (b) together with the following component (c), and the pH value thereof is adjusted to 8 to 11 at the use, in order to improve the effects of the aforesaid hair treatment composition:
(c) one or more compounds selected from the group consisting of ammonia, amino compounds, amines and quaternary ammonium salts.

Furthermore, the present invention provides a hair treatment composition which comprises:
(a) one or more compounds represented by the above general formula (I) or (II);
(b) one or more compounds represented by the above general formula (III) or (IV); and
(d) one or more oxidizing agents.

The present invention still provides a cyclic Bunte salt-modified silicone represented by the following general formula (V): 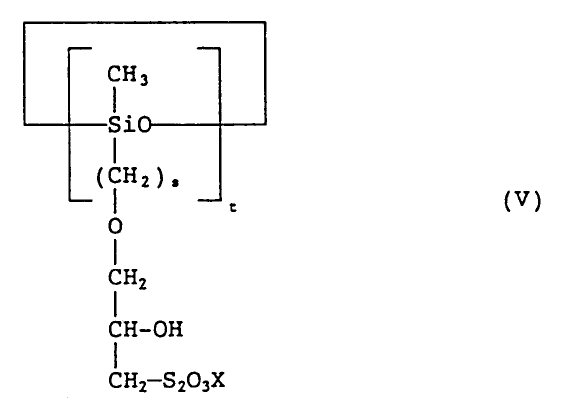 wherein X represents an alkali metal, an alkaline earth metal, an ammonium, a primary ammonium, a secondary ammonium, a tertiary ammonium or a quaternary ammonium; t is an integer of from 4 to 10; and s is an integer of from 3 to 11.

The present invention still further provides a hair treatment composition which comprises one or more cyclic Bunte salt-modified silicones represented by the above general formula (V), and the pH value thereof is adjusted to 2 to 4 or 8 to 11 at the use.

The present invention still furthermore provides a hair treatment composition which comprises:
(e) one or more cyclic Bunte salt-modified silicones represented by the above general formula (V); and
(d) one or more oxidizing agents.

The present invention yet furthermore provides a hair treatment composition which comprises:
(e) one or more cyclic Bunte salt-modified silicones represented by the above general formula (VI); and
(f) one or more reducing agents.

### DETAILED DESCRIPTION OF THE INVENTION

Now the hair treatment composition of the present invention will be described in detail.

Examples of the alkali metal represented by X in the compound of the above general formula (I) or (II) (hereinafter referred to as "Silicone component (a)") include sodium, potassium and lithium. Examples of the alkaline earth metal therein include magnesium and calcium. Examples of the primary, secondary and tertiary ammonium therein include groups of monoethanolamine, diethanolamine, triethanolamine and amino acids. Examples of the quaternary ammonium therein include mono- or di-long chain alkyl quaternary ammonium having straight-chain or branched alkyl group(s) and polyoxyalkylene-adducts thereof.

As the straight-chain or branched alkyl group, those having 7 to 32 carbon atoms are preferred and those having 7 to 22 carbon atoms are more preferred.

As the alkylene group in the polyoxyalkylene, those having 2 to 6 carbon atom are preferred and those having 2 to 4 carbon atoms are more preferred. The polymerization degree of the polyoxyalkylene is preferably from 1 to 2,000.

Specific examples of the quaternary ammonium include cetyltrimethylammonium, stearyltrimethylammonium, behenyltrimethylammonium and laurylmyristyldimethylammonium.

The aforesaid Silicone component (a) may be synthesized by, for example, a method described in West German Patent No. 3735086.

The content of the aforesaid Silicone component (a) in the composition may preferably range from 0.01 to 50 % by weight, more preferably from 0.1 to 20 % by weight, based on the total weight of the composition. As Silicone component (a), one or more compounds represented by the above general formula (I) or those represented by the above general formula (II) may be used. It is preferable to use both of the compounds of the general formulae (I) and (II) in combination thereof. The weight ratio of the compound (I)/the compound (II) may preferably range from 1/10 to 10/1, more preferably from 1/5 to 5/1. The balance of softness/hardness of a coating film formed on the surface of the hair may be controlled by varying this ratio. In general, the hardness of the coating film is elevated with an increase in the content of the compound of the general formula (I).

The properties of the aforesaid coating film may be controlled by varying n, m and ℓ in the general formula (I) or (II). It is preferable that n, m and ℓ are at least 4. In the compound represented by the above general formula (I), it is preferable that the units thereof are polymerized each other in a block or random state and that the ratio of n/m is at least 2.

The molecular weight (Mw) of the compound of formula (I) or (II) is preferably 1,000 or more.

Examples of the compound represented by the above general formula (III) used in the present invention include isopropyl alcohol, ethyl alcohol and benzyl alcohol. It is particularly preferable to use ethyl alcohol or benzyl alcohol.

As the compound represented by the above general formula (IV) used in the present invention, those wherein R₃ is an alkyl group having 8 to 12 carbon atoms are preferable.

The content of the compound represented by the above general formula (III) or (IV) (hereinafter referred to as "Component (b)") in the composition may preferably range from 0.1 to 50 % by weight, more preferably from 0.5 to 50 % by weight, based on the total weight of the composition.

The aforesaid Component (b) can extremely enhance the adsorption and penetration of Silicone component (a) into the hair. In the case of the hair damaged at the end or the permed hair, in particular, it can exert a sustained effect of preventing hair from causing a split hair and imparts a gloss through adsorption and penetration.

In the hair treatment composition of the present invention, the weight ratio of Silicone Component (a) to Component (b) ((a)/(b)) may preferably range from 1/20 to 20/1, more preferably from 1/10 to 10/1.

Examples of the amino compound of the component (c) used in the present invention (hereinafter referred to as "Component (c)") include cysteine, arginine, glycine and glutamic acid. Examples of the amine thereof include triethanolamine, diethanolamine and monoethanolamine. Examples of the quaternary ammonium salt thereof include stearyltrimethylammonium chloride, mono- or di-long chain alkyl quaternary ammonium salts having straight-chain or branched alkyl group(s), and polyoxyalkylene-adducts thereof.

As the straight-chain or branched alkyl group, those having 7 to 32 carbon atoms are preferred and those having 7 to 22 carbon atoms are more preferred.

As the alkylene group in the polyoxyalkylene, those having 2 to 6 carbon atom are preferred and those having 2 to 4 carbon atoms are more preferred. The polymerization degree of the polyoxyalkylene is preferably from 1 to 2,000.

Specific examples of the quaternary ammonium salt include cetyltrimethylammonium salt, stearyltrimethylammonium salt, behenyltrimethylammonium salt and laurylmyristyldimethylammonium salt.

As aforesaid Component (c), ammonia and amines are particularly preferable.

The content of Component (c) in the composition may preferably range from 0.1 to 10 % by weight, more preferably from 0.5 to 5 % by weight, based on the total weight of the composition.

The pH value of the hair treatment composition of the present invention is adjusted to 2 to 4 or 8 to 11, preferably to 2 to 3.5 or 8 to 10, at the use. When the pH value of the hair treatment composition is smaller than 2 or exceeds 11, there is tendency to irritate the skin or damage the hair. When it falls within a range of from 4 to 8, on the other hand, neither the effect of preventing hair from causing a split hair nor the effect of improving hair gloss sustained for a long period of time can be achieved.

The pH value of the hair treatment composition of the present invention may be adjusted by using an acidic solution containing, for example, an organic acid such as citric acid, lactic acid, tartaric acid, malic acid, succinic acid, gluconic acid or an inorganic acid such as hydrochloric acid, sulfuric acid, nitric acid; or an alkaline solution containing, for example, hydroxide, acetate or hydrogen-carbonate of an alkali metal such as sodium, potassium, lithium or an alkaline earth metal such as magnesium or calcium. In the case of a hair treatment composition comprising ammonia, triethanolamine or monoethanolamine as Component (c), the pH value may be adjusted by controlling the content of these compounds. When the pH value of the hair treatment composition of the present invention already falls within the range as specified above, therefore, it is not required to adjust its pH value any more.

The hair treatment composition of the present invention may contain oxidizing agent(s) as Component (d). In this case, any adjustment of the pH value is not required.

Examples of the oxidizing agent to be used in the present invention include peroxides such as hydrogen peroxide, sodium percarbonate and sodium perborate and bromates such as sodium bromate and potassium bromate. It is particularly preferable to use hydrogen peroxide and sodium bromate.

The content of the aforesaid oxidizing agent in the composition may preferably range from 0.05 to 30 % by weight, more preferably from 0.5 to 10 % by weight, based on the total weight of the composition.

The hair treatment composition of the present invention which comprises Silicone component (a) and Component (b); the hair treatment composition of the present invention which comprises Silicone component (a), Component (b) and Component (c); and the hair treatment composition of the present invention which comprises Silicone component (a), Component (b) and the aforesaid oxidizing agent (d), may each further contain components commonly used in hair cosmetics, for example, surfactant, polymer compound, oily component, polyhydric alcohol and other components, depending on the purpose, usage and form of the aimed product, so long as the effects of the present invention are not deteriorated thereby. Water is contained in the hair treatment composition of the present invention in the balance amount.

Examples of the aforesaid surfactant include straight-chain or branched alkyl benzenesulfonates, ethylene oxide and/or propylene oxide-added alkyl or alkenyl ether sulfates, olefin sulfonates, alkaline sulfonates, saturated or unsaturated fatty acid salts, ethylene oxide and/or propylene oxide-added alkyl or alkenyl ether carboxylates, α-sulfo fatty acid salt ester, amino acid surfactants, sulfosuccinic acid surfactants, phosphate surfactants, sulfonate ampholytic surfactants, betaine ampholytic surfactants, alkylamine oxides, cationic surfactants, polyoxyalkyl or alkenyl ethers, polyoxyalkyl phenyl ethers, higher fatty acid alkanolamide or alkylene oxide adducts thereof, polyhydric alcohol/fatty acid esters, sorbitol/fatty acid esters, sucrose/fatty acid esters and higher alcohol/sugar ethers. Examples of the aforesaid polymer compound include cationized cellulose derivatives, cationic starch, cationized guar gum derivatives, diallyl quaternary ammonium salt/acrylamide copolymers, quaternarized polyvinylpyrrolidone derivatives and polyglycolamine condensates, carboxyvinyl polymer, xanthan gum, methylcellulose and hydroxyethylcellulose. Examples of the aforesaid oily component include higher fatty acids such as stearic acid; higher alcohols such as cetanol; solid fats such as cholesterol, vaseline and cholesteryl isostearate; liquid oils such as squalene and jojoba oil; and silicone derivatives other than the aforesaid Silicone Component (a) used in the present invention. Examples of the aforesaid polyhydric alcohol include glycerol, 1,3-butylene glycol, propylene glycol, dipropylene glycol, polyethylene glycol and sorbitol. Examples of the aforesaid other components include various medical components, chelating agents, pearling agents, perfumes, colorants, UV absorbers, antioxidant, bactericides such as triclosan and trichlorocarban, antiinflammatory agents such as potassium glycyrrhetinate and tocopherol acetate, anti-dandruff agents such as zinc pyrithione and piroctone olamine, and preservatives such as methylparaben and butylparaben.

The hair treatment composition of the present invention may be usually provided in the form of, for example, a two-pack type composition consisting of:
the first pack comprising Silicone Component (a); or Silicone Component (a) and Component (b); and
the second pack comprising Component (b) and one of Component (c), the oxidizing agent (d) and, if required, the acidic or alkaline solution for adjusting the pH value of the whole composition within the range as specified above; or one of Component (c), the oxidizing agent (d) and, if required, the acidic or alkaline solution.

Either one of the first or second pack is applied to the hair followed by another one. Alternately, the first and second packs are mixed together immediately before the use and then the resulting mixture is applied to the hair.

Next, the cyclic Bunte salt-modified silicone of the present invention and another embodiments of the hair treatment composition of the present invention containing the same will be described in detail.

Examples of the alkali metal of X in the cyclic Bunte salt-modified silicone of the present invention represented by the aforesaid general formula (V) (hereinafter referred to as the "cyclic silicone (e)") include sodium, potassium and lithium. Examples of the alkaline earth metal therein include magnesium and calcium. Examples of the primary, secondary and tertiary ammonium therein include group of monoethanolamine, diethanolamine, triethanolamine and amino acids. Examples of the quaternary ammonium therein include mono- or di-long chain alkyl quaternary ammonium having straight-chain or branched alkyl group(s) and polyoxyalkylene adducts thereof.

As the straight-chain or branched alkyl group, those having 7 to 32 carbon atoms are preferred and those having 7 to 22 carbon atoms are more preferred.

As the alkylene group in the polyoxyalkylene, those having 2 to 6 carbon atom are preferred and those having 2 to 4 carbon atoms are more preferred. The polymerization degree of the polyoxyalkylene is preferably from 1 to 2,000.

Specific examples of the quaternary ammonium include cetyltrimethylammonium, stearyltrimethylammonium, behenyltrimethylammonium and laurylmyristyldimethylammonium.

The aforesaid cyclic silicone may be produced by, for example, the following method.

First, monomethylcyclopolysiloxane is reacted with allyl glycidyl ether.

This reaction may be catalyzed by platinum chloride, a platinum catalyst carried on, for example, carbon or alumina, a platinum/olefin complex or a platinum/phosphine complex. It may also be catalyzed by a complex of a transition metal of the group VIII. This reaction may be performed in the presence of one of these catalyst at a temperature of from room temperature to approximately 200°C for 0.5 to 10 hours. In this reaction, monomethylcyclopolysiloxane and allyl glycidyl ether may be preferably used at a molar ratio of from 1 : 1 to 1 : 10.

Next, the reaction product thus obtained is treated with a thiosulfate to thereby open the epoxy ring of the reaction product.

This ring-opening reaction may be performed by treating the reaction product with the thiosulfate in the presence of a phase transfer catalyst, (e.g., a compound represented by the following general formula (VI)) by using a solvent such as a water/ethanol solvent mixture or a water/isopropanol solvent mixture at a temperature of from room temperature to the reflux point for 0.5 to 24 hours. During the reaction period, the pH value of the reaction mixture would be elevated. It is preferable, therefore, that the reaction is performed while controlling the pH value of the reaction mixture within 5 to 9 by using, for example, a solution of HCl, H₂SO₄ or H₃PO₄. The molar ratio of the aforesaid reaction product to the aforesaid thiosulfate may be preferably adjusted to 1 : 1 to 1 : 3.

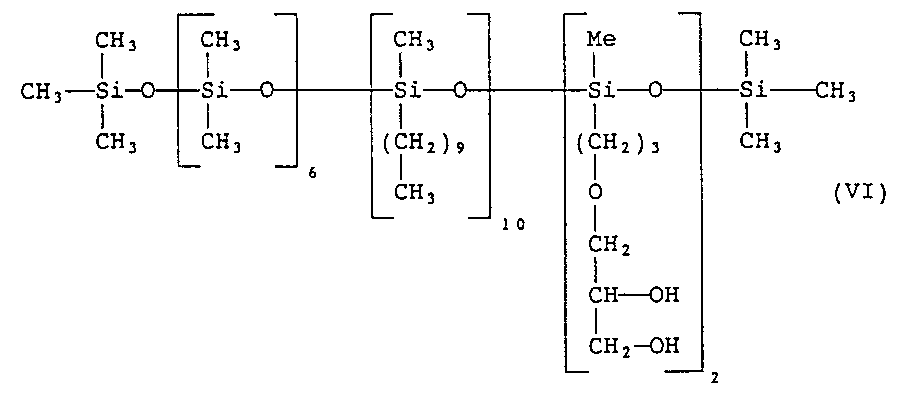

Thus the cyclic Bunte salt-modified silicone of the present invention represented by the general formula (V) may be obtained.

The compound of the general formula (VI) may be synthesized by the method as disclosed, for example, in Japanese Patent Application No. Hei-2-228958.

The cyclic silicone (e) of the present invention is a white to pale yellow solid which decomposes at 150°C.

The aforesaid cyclic silicone (e) of the present invention is useful as a conditioning component to be used in a hair treatment composition (for example, shampoo, rinse, treatment, styling agent, permanent waving agent, hair color, bleach). In addition, it is useful as, for example, a surface treatment agent for textile, fiber and paper.

In these cases, the molecular weight (Mw) of the cyclic silicone (e) is preferably 1,000 or more.

As the aforesaid cyclic silicone (e), those represented by the general formula (V) wherein t is from 4 to 6 are preferable.

The content of the aforesaid cyclic silicone in a hair treatment composition may preferably range from 0.01 to 50 % by weight, more preferably from 0.1 to 20 % by weight, based on the total weight of the composition.

Furthermore, the hair treatment composition of the present invention preferably contain one or more compounds represented by the following general formula (III) or (IV) as Component (b): 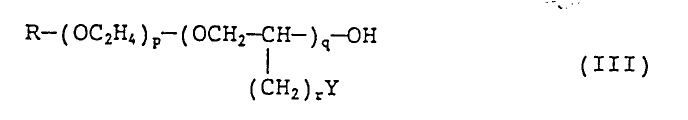 wherein R represents a hydrogen atom or an 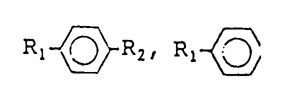 or a straight-chain or branched alkyl group having 1 to 4 carbon atoms, in which R₁ represents a hydrogen atom, a methyl group or a methoxy group and R₂ represents a -CH₂-, -C(CH₃)₂-, -CH₂-CH(CH₃)- or -CH=CH-CH₂- group; Y represents a hydrogen atom or a hydroxyl group; and p, q and r are each an integer of from 0 to 5, provided that at least one of p and q is 0j and provided that in the compound of formula (III) p and q may not be 0 at the same time, when R hydrogen; 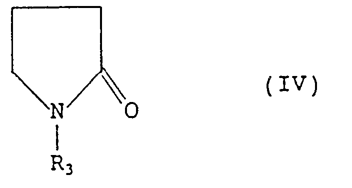 wherein R₃ represents a straight-chain or branched alkyl group having 8 to 18 carbon atoms.

Examples of the compound represented by the above general formula (III) include isopropyl alcohol, ethyl alcohol and benzyl alcohol. Among these compounds, ethyl alcohol and benzyl alcohol are particularly preferable therefor.

As the compound represented by the above general formula (IV), those wherein R₃ is an alkyl group having 8 to 12 carbon atoms are preferable.

The content of the compound represented by the above general formula (III) or (IV) (Component (b)) in the composition may preferably range from 0.1 to 50 % by weight, more preferably from 0.5 to 50 % by weight, based on the total weight of the composition.

The compound represented by the above general formula (III) or (IV) (Component (b)) can extremely enhance the adsorption and penetration of the aforesaid cyclic silicone into the hair. In the case of the hair damaged at the end or the permed hair, in particular, it can exert a sustained effect of preventing hair from causing a split hair and impart improved gloss to the hair through adsorption and penetration.

In the hair treatment composition of the present invention, the weight ratio of the cyclic silicone (e) of the general formula (V) to Component (b) ((e)/(b)) may preferably range from 1/20 to 20/1, more preferably from 1/10 to 10/1.

Examples of the amino compound of the compounds of the component (c) used in the present invention (hereinafter referred to as "Component (c)") include cysteine, arginine, glycine and glutamic acid. Examples of the amine thereof include triethanolamine, diethanolamine and monoethanolamine. Examples of the quaternary ammonium salt thereof include stearyltrimethylammonium chloride, mono- or di-long chain alkyl quaternary ammonium salts having straight-chain or branched alkyl group(s), and polyoxyalkylene-adducts thereof.

As the straight-chain or branched alkyl group, those having 7 to 32 carbon atoms are preferred and those having 7 to 22 carbon atoms are more preferred.

As the alkylene group in the polyoxyalkylene, those having 2 to 6 carbon atom are preferred and those having 2 to 4 carbon atoms are more preferred. The polymerization degree of the polyoxyalkylene is preferably from 1 to 2,000.

Specific examples of the quaternary ammonium salt include cetyltrimethylammonium salt, stearyltrimethylammonium salt, behenyltrimethylammonium salt and laurylmyristyldimethylammonium salt.

As aforesaid Component (c), ammonia and amines are particularly preferable.

The content of Component (c) in the composition may preferably range from 0.1 to 10 % by weight, more preferably from 0.5 to 5 % by weight, based on the total weight of the composition.

The pH value of the hair treatment composition of the present invention is adjusted to from 2 to 4, preferably from 2 to 3.5, or from 8 to 11, preferably from 8 to 10, at the use. When the pH value of the hair treatment composition falls within a range of from 4 to 8, neither the effect of preventing hair from causing a split hair nor the effect of improving hair gloss sustained for a long period of time can be achieved. When the pH value thereof is smaller than 2 or exceeds 11, on the other hand, there is a tendency to irritate the skin or damage the hair.

The pH value of the hair treatment composition of the present invention may be adjusted to 2 to 4 by using an acidic solution containing, for example, an organic acid such as citric acid, lactic acid, tartaric acid, malic acid, succinic acid or gluconic acid or an inorganic acid such as hydrochloric acid, sulfuric acid or nitric acid. On the other hand, the pH value thereof may be adjusted to 8 to 11 by using an alkaline solution containing, for example, hydroxide, acetate or hydrogencarbonate of an alkali metal such as sodium, potassium or lithium or an alkaline earth metal such as magnesium or calcium. In the case of a hair treatment composition which contains ammonia, triethanolamine or monoethanolamine as an arbitrary component, the pH value may be adjusted by controlling the content of these compounds. When the pH value of the hair treatment composition of the present invention already falls within the range as specified above, therefore, it is not required to adjust its pH value any more.

The hair treatment composition of the present invention may contain an oxidizing agent (d) or a reducing agent (f). In these cases, any adjustment of the pH value is not required.

Examples of the oxidizing agent (d) to be used in the present invention include peroxides such as hydrogen peroxide, sodium percarbonate and sodium perborate and bromates such as sodium bromate and potassium bromate. It is particularly preferable to use hydrogen peroxide and sodium bromate.

The content of the aforesaid oxidizing agent (d) in the composition may preferably range from 0.05 to 30 % by weight, more preferably from 0.5 to 10 % by weight, based on the total weight of the composition.

Examples of the reducing agent (f) to be used in the present invention include thioglycolic acid and thioglycolates (preferable examples of thioglycolate include ammonium thioglycolate, alkali metal thioglycolates such as sodium and potassium thioglycolates and alkaline earth metal thioglycolates such as calcium thioglycolate), cysteine and salts thereof (a preferable example of cysteine salt is cysteine hydrochloride) and L-acetylcysteine and salts thereof (a preferable example of L-acetylcysteine salt is L-acetylcysteine hydrochloride). Among these compounds, thioglycolic acid, ammonium thioglycolate, cysteine and cysteine hydrochloride are particularly preferable therefor.

The content of the aforesaid reducing agent (f) in the composition may preferably range from 0.1 to 20 % by weight, still preferably from 0.5 to 10 % by weight, based on the total weight of the composition.

The hair treatment composition of the present invention may further contain ammonia, amino compounds, amines and quaternary ammonium salts, if desired. Examples of the aforesaid amino compound include cysteine, arginine, glycine and glutamic acid. Examples of the aforesaid amine include triethanolamine, diethanolamine and monoethanolamine. Examples of the quaternary ammonium salt include mono- or di-long chain alkyl quaternary ammonium salts having straight-chain or branched alkyl group(s) such as stearyltrimethylammonium chloride and polyoxyalkylene-adducts thereof.

The hair treatment composition of the present invention may further contain components commonly used in hair cosmetics, for example, surfactant, polymer compound, oily component, polyhydric alcohol and other components, depending on the purpose, usage and form of the aimed product, so long as the effects of the present invention are not deteriorated thereby. Water is contained in the hair treatment composition of the present invention in the balance amount.

Examples of the aforesaid surfactant include straight-chain or branched alkyl benzenesulfonates, ethylene oxide and/or propylene oxide-added alkyl or alkenyl ether sulfates, olefin sulfonates, alkane sulfonates, saturated or unsaturated fatty acid salts, ethylene oxide and/or propylene oxide-added alkyl or alkenyl ether carboxylates, α-sulfo fatty acid salt ester, amino acid surfactants, sulfosuccinic acid surfactants, phosphate surfactants, sulfonate ampholytic surfactants, betaine ampholytic surfactants, alkylamine oxides, cationic surfactants, polyoxyalkyl or alkenyl ethers, polyoxyalkyl phenyl ethers, higher fatty acid alkanolamide or alkylene oxide adducts thereof, polyhydric alcohol/fatty acid esters, sorbitol/fatty acid esters, sucrose/fatty acid esters and higher alcohol/sugar ethers. Examples of the aforesaid polymer compound include cationized cellulose derivatives, cationic starch, cationized guar gum derivatives, diallyl quaternary ammonium salt/acrylamide copolymers, quaternarized polyvinylpyrrolidone derivatives and polyglycolamine condensates, carboxyvinyl polymer, xanthan gum, methylcellulose and hydroxyethylcellulose. Examples of the aforesaid oily component include higher fatty acids such as stearic acid; higher alcohols such as cetanol; solid fats such as cholesterol, vaseline and cholesteryl isostearate; liquid oils such as squalene and jojoba oil; and silicone derivatives other than the aforesaid cyclic silicone used in the present invention. Examples of the aforesaid polyhydric alcohol include glycerol, 1,3-butylene glycol, propylene glycol, dipropylene glycol, polyethylene glycol and sorbitol. Examples of the aforesaid other components include various medical components, chelating agents, pearling agents, perfumes, colorants, UV absorbers, antioxidant, bactericides such as triclosan and trichlorocarban, antiinflammatory agents such as potassium glycyrrhetinate and tocopherol acetate, anti-dandruff agents such as zinc pyrithione and piroctone olamine, and preservatives such as methylparaben and butylparaben.

The hair treatment composition of the another embodiment of the present invention may be usually provided in the form of, for example, a two-pack type composition consisting of:
the first pack comprising the cyclic silicone (e); or the cyclic silicone (e) and Component (b); and
the second pack comprising the Component (b) and one of Component (C), the oxidizing agent (d), the reducing agent (f) and, if required, the acidic or alkaline solution for adjusting the pH value of the whole composition within the range as specified above; or one of Component (c), the oxidizing agent (d), the reducing agent (f) and, if required, the acidic or alkaline solution.

Either one of the first or second pack is applied to the hair followed by another one. Alternately, the first and second packs are mixed together immediately before the use and then the resulting mixture is applied to the hair.

The hair treatment composition of the present invention may be used as, for example, shampoo, rinse, treatment, styling agent, permanent waving agent, hair color, bleach.

The hair treatment composition of the present invention may be applied to the hair and dried as such. Alternately, the hair may be rinsed and then dried. Then the conditioning components of the hair treatment composition are attached to the damaged part of the hair and penetrates therein. Thus a coating film, which can sustain for a long period of time, is formed on the surface of the hair and an effect of preventing hair from causing a split hair and an effect of improving hair gloss can be exerted on the hair for a long time.

To further illustrate the present invention, and not by way of limitation, the following Examples and Comparative Examples will be given.

### EXAMPLE 1

Two-pack system compositions (Products of the Invention 1-1 to 1-4 and Comparative Products 1-1 and 1-2) of the formulations as specified in Table 1 were prepared. About 20 g of each composition was applied to about 20 g of the permed black hair of a Japanese subject.

Table 1 shows the effects thus achieved.

### EXAMPLE 2

Two-pack system compositions (Products of the Invention 1-5 to 1-8 and Comparative Products 1-3 and 1-4) of the formulations as specified in Table 2 were prepared. About 20 g of each composition was applied to about 20 g of the permed black hair of a Japanese subject.

Table 2 shows the effects thus achieved.

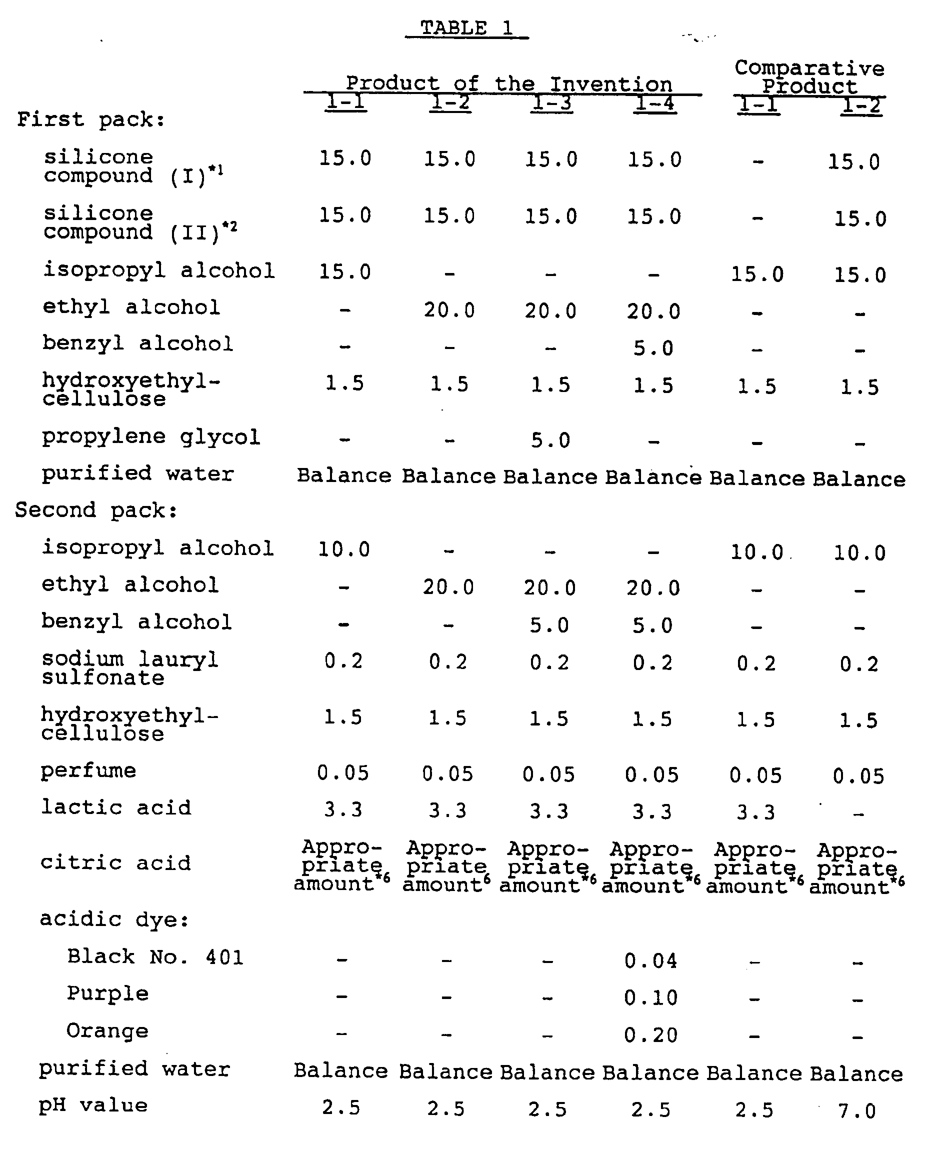

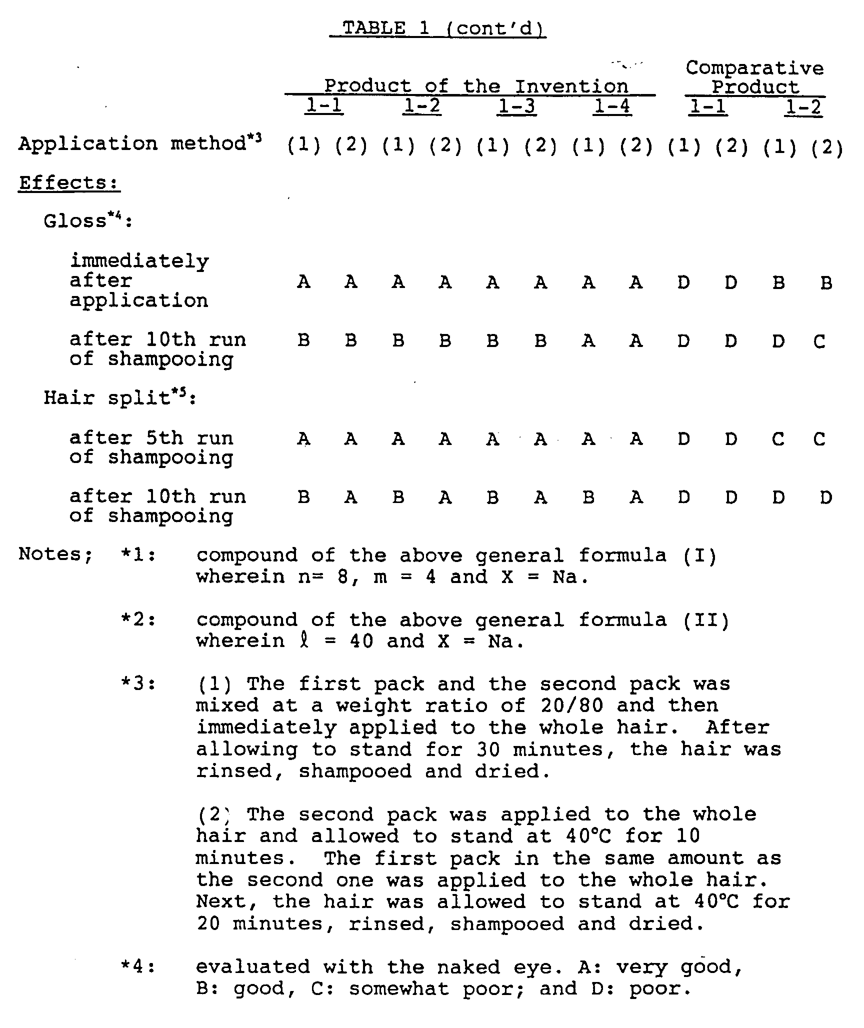

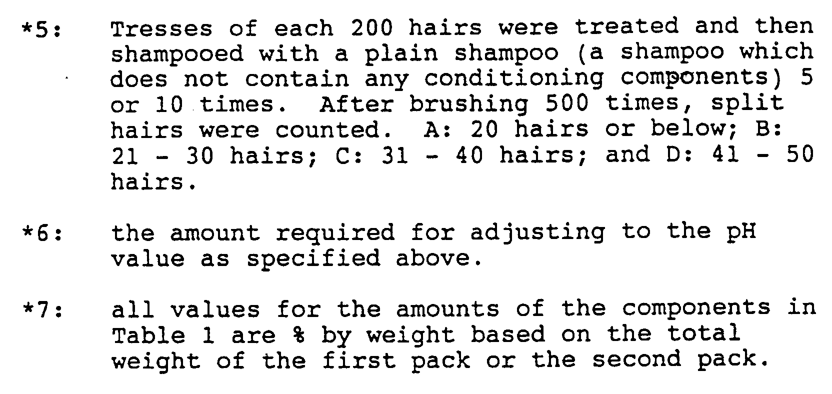

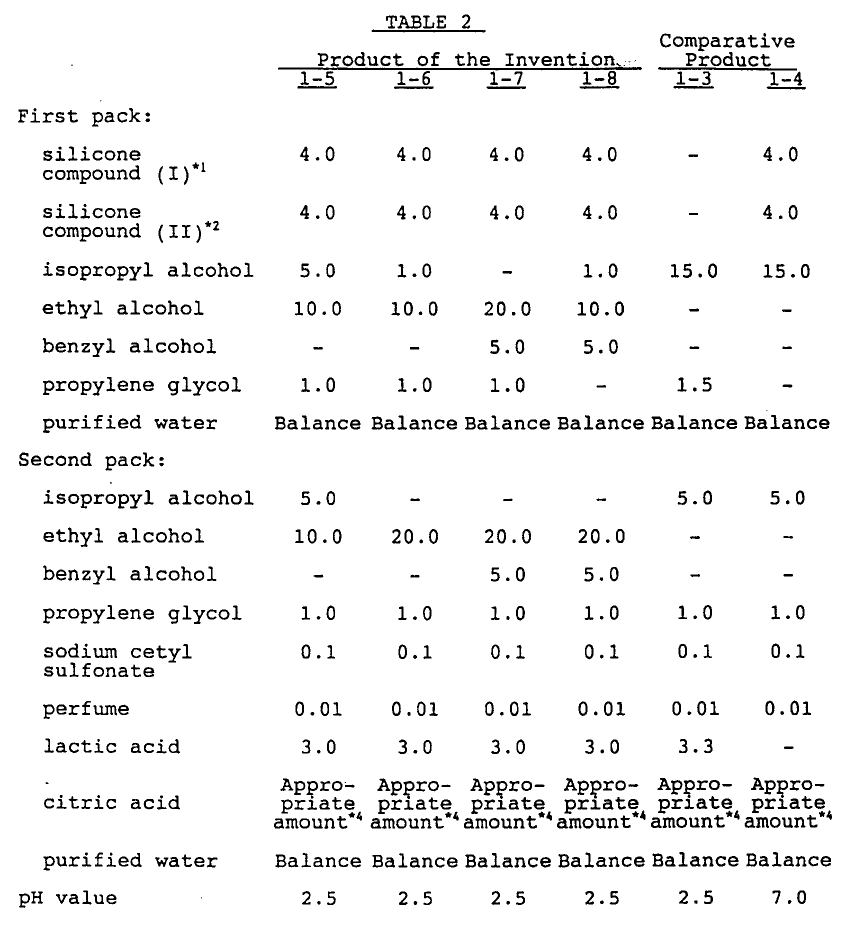

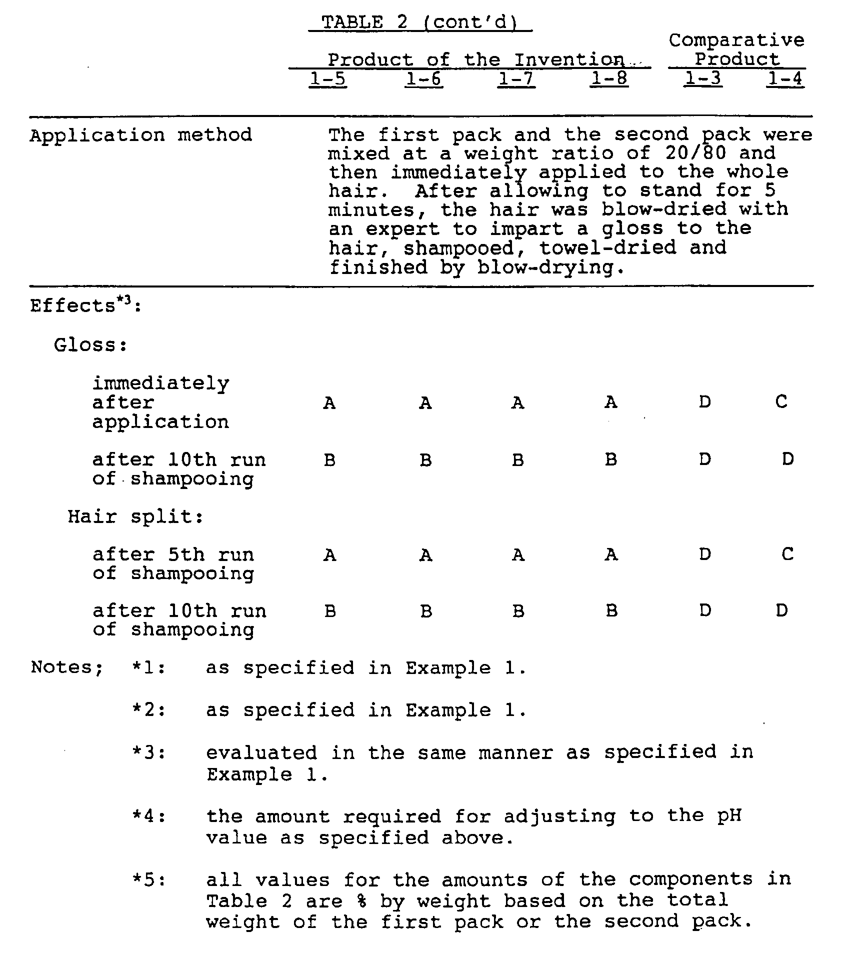

### EXAMPLE 3

Two-pack system compositions (Products of the Invention 2-1 to 2-4 and Comparative Products 2-1 and 2-2) of the formulations as specified in Table 3 were prepared. About 20 g of each composition was applied to about 20 g of the permed black hair of a Japanese subject.

Table 3 shows the effects thus achieved.

### EXAMPLE 4

Two-pack system compositions (Products of the Invention 2-5 to 2-7 and Comparative Products 2-3 and 2-4) of the formulations as specified in Table 4 were prepared. About 20 g of each composition was applied to about 20 g of the permed black hair of a Japanese subject.

Table 4 shows the effects thus achieved.

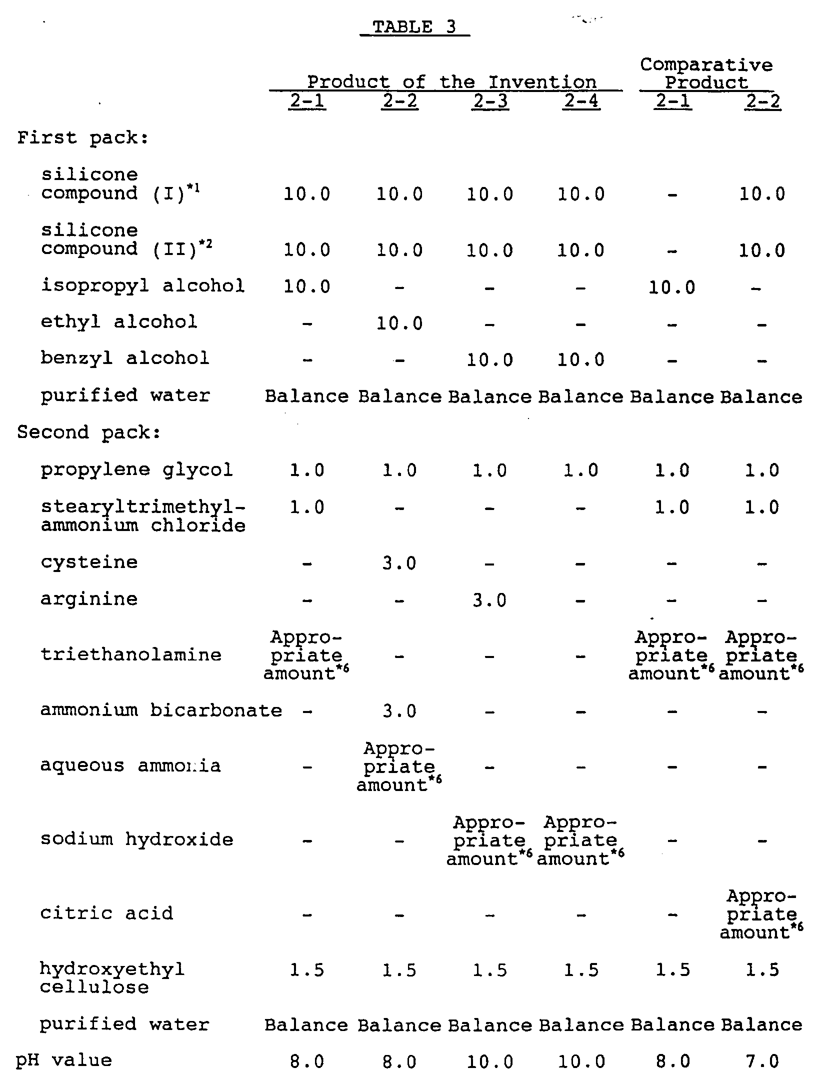

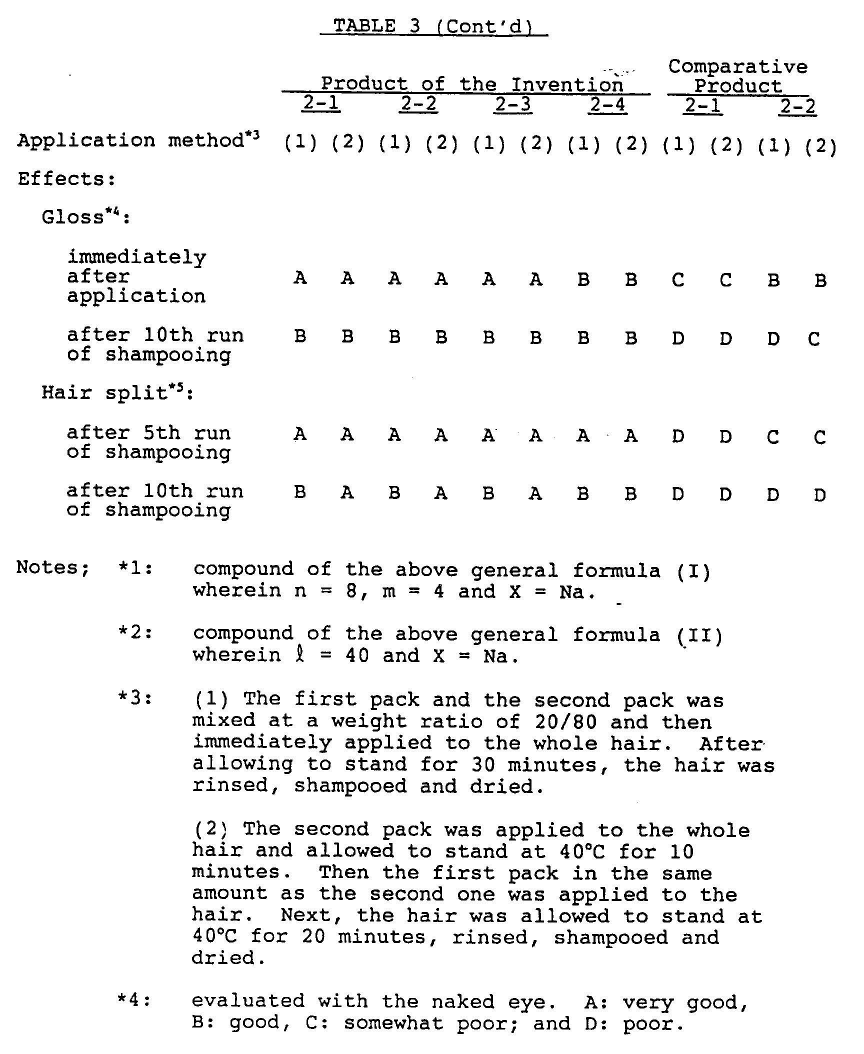

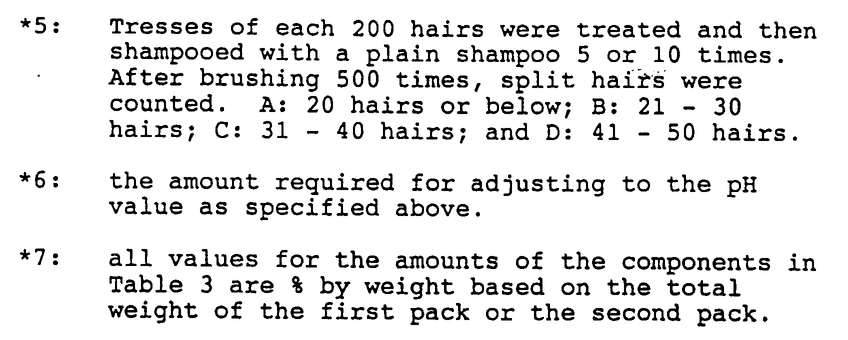

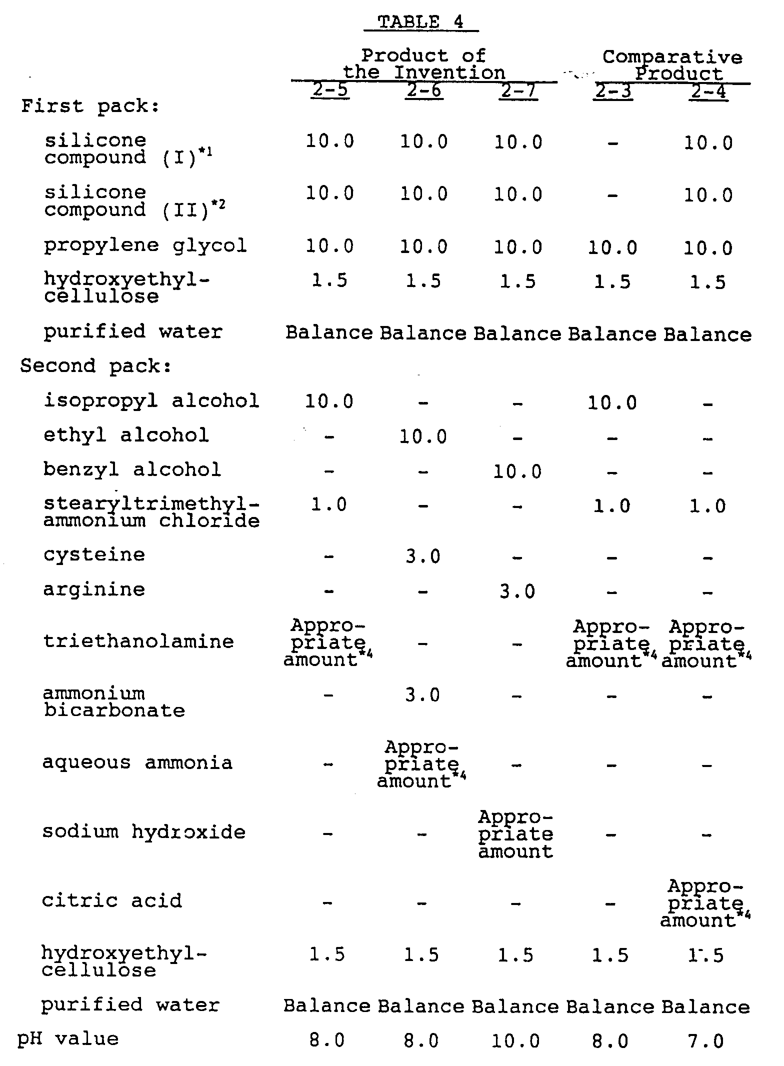

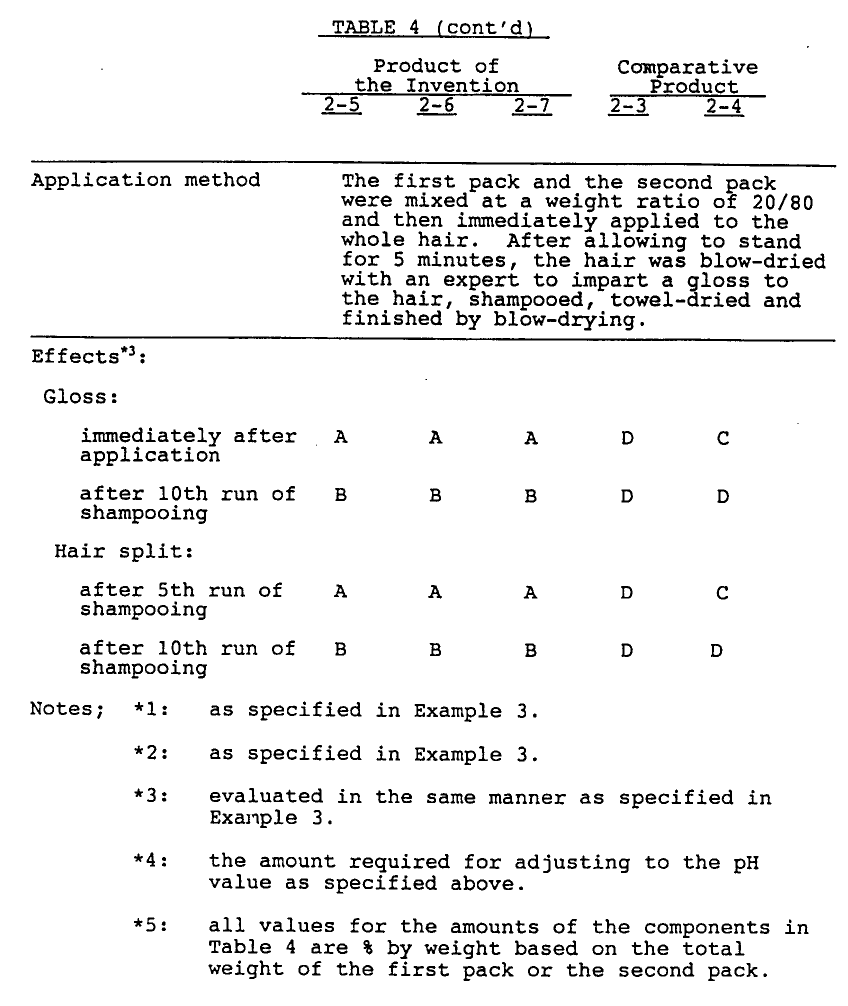

### EXAMPLE 5

Two-pack system compositions (Products of the Invention 3-1 to 3-3 and Comparative Products 3-1 and 3-2) of the formulations as specified in Table 5 were prepared. About 20 g of each composition was applied to about 20 g of the permed black hair of a Japanese subject.

Table 5 shows the effects thus achieved.

### EXAMPLE 6

Two-pack system compositions (Products of the Invention 3-4 to 3-6 and Comparative Products 3-3 and 3-4) of the formulations as specified in Table 6 were prepared. About 20 g of each composition was applied to about 20 g of the permed black hair of a Japanese subject.

Table 6 shows the effects thus achieved.

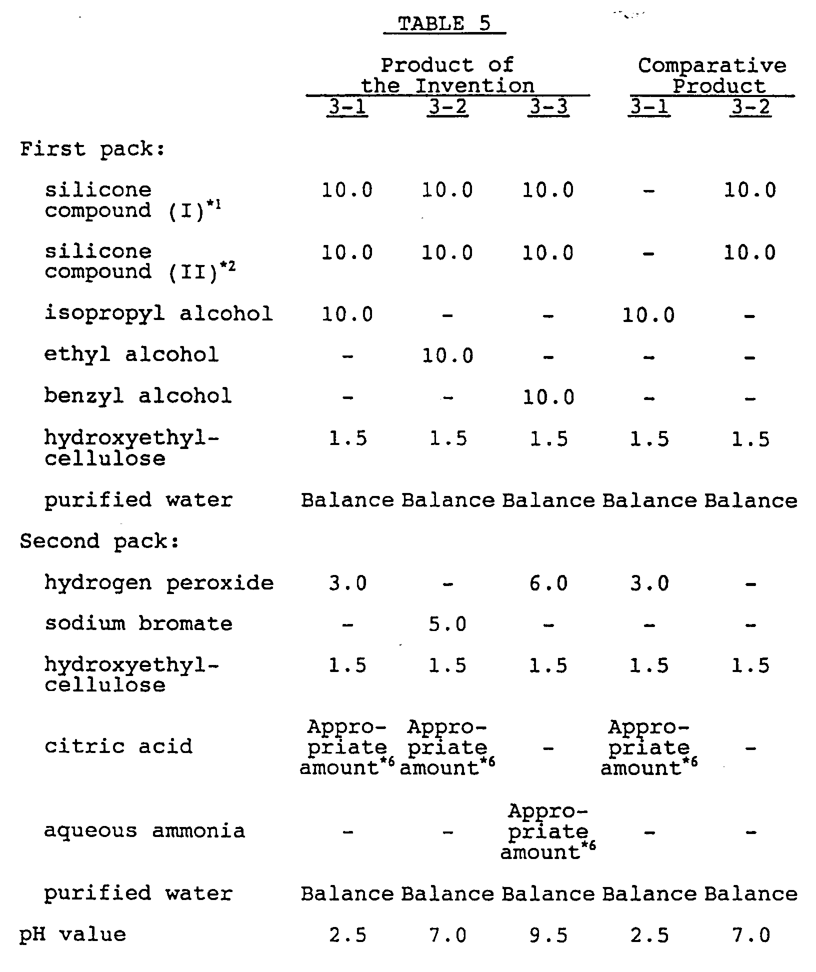

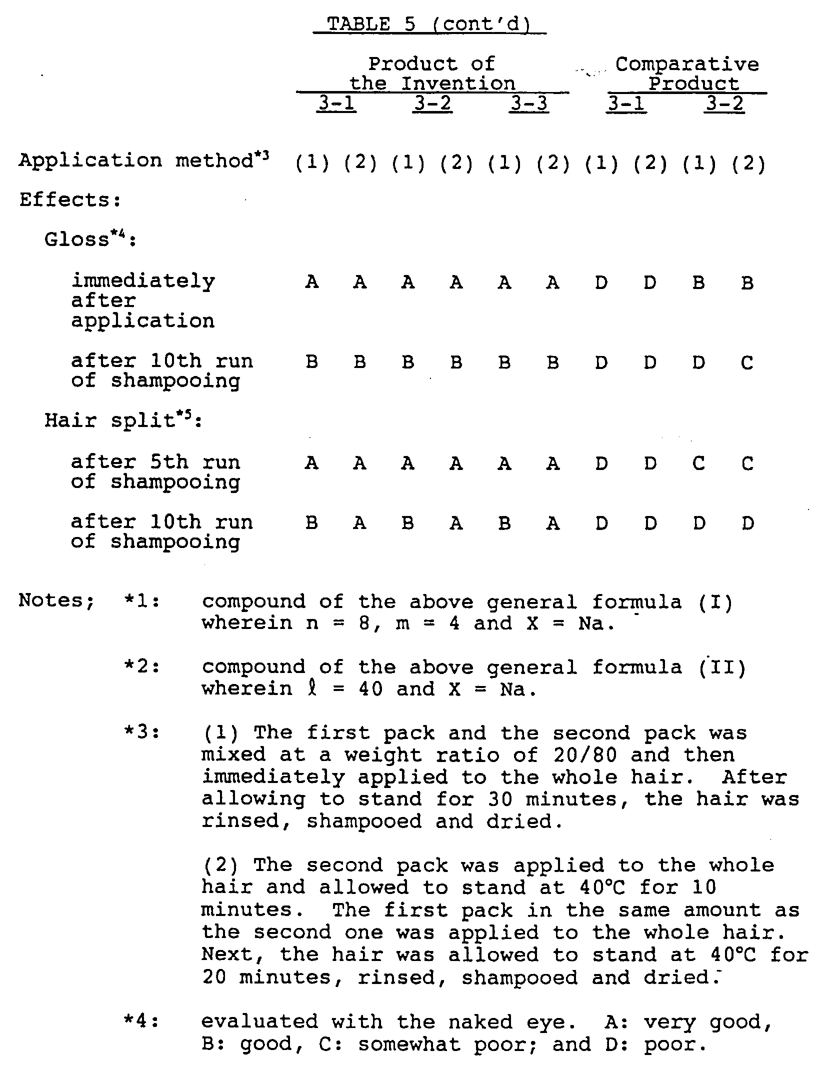

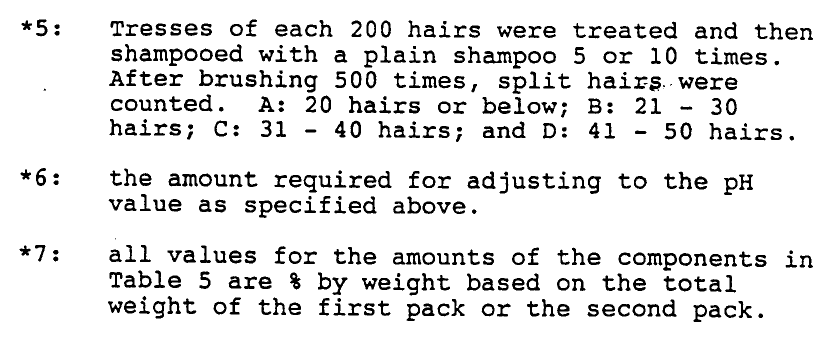

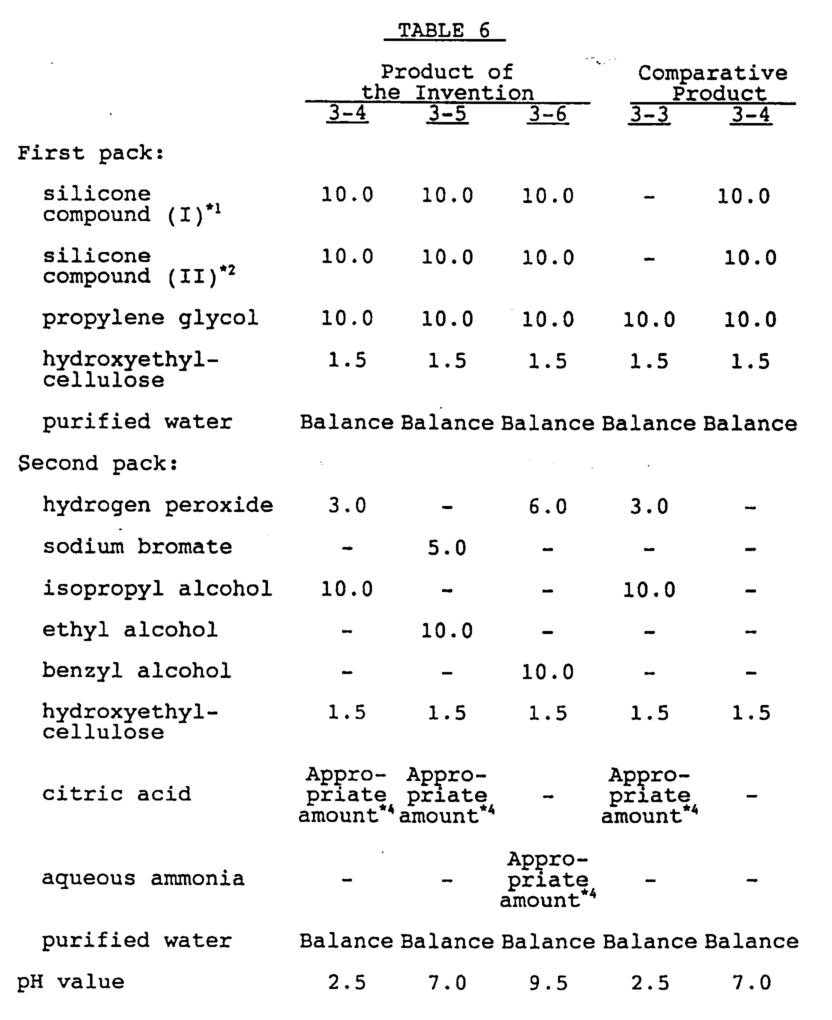

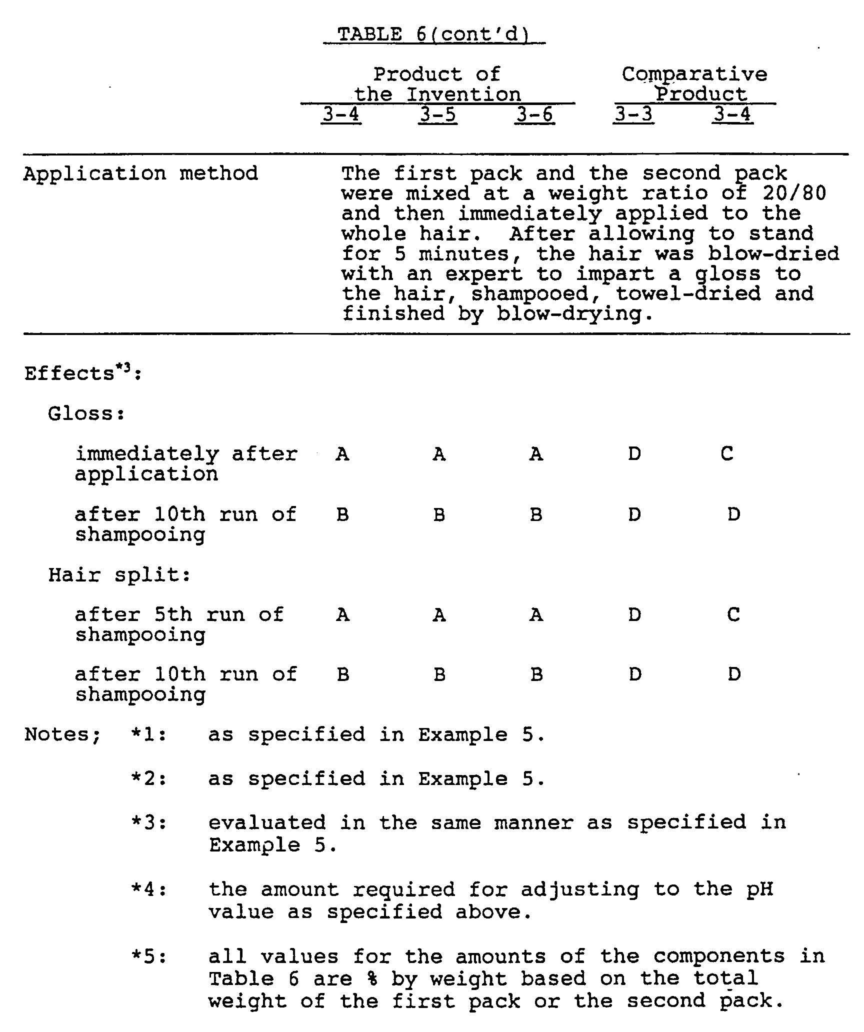

### EXAMPLE 7

### Synthesis of cyclic silicone of the general formula (V) wherein n = 4, m = 3 and X = Na:

(1) 50 g of tetramethylcyclotetrasiloxane and 123.4 g of allyl glycidyl ether were fed into a 300 ml three-neck flask. Then 30 µl of a 2 % isopropanol solution of platinic chloride in was added thereto under thoroughly stirring. After 2 hours, the excessive allyl glycidyl ether was distilled off from the reaction mixture under reduced pressure. Thus 120 g of a colorless transparent liquid was obtained. This product was a silicone compound represented by the following formula (VII). 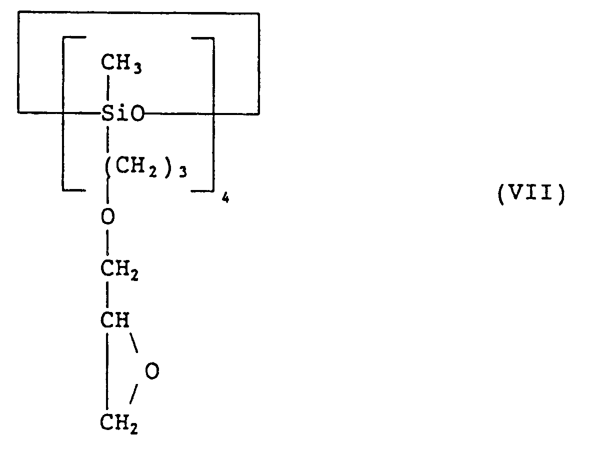
(2) 15 g of the silicone compound obtained in the above (1), 21.4 g of Na₂S₂O₃·5H₂O, 35.2 g of water, 60.6 g of ethanol, 0.5 g of a 0.1 % phenolphthalein solution and 0.4 g of a compound represented by the formula (VI) were fed into a 300 ml three-neck flask and heated to 80°C under stirring. During the reaction period, the pH value of the mixture was elevated. Thus the reaction was allowed to proceed while neutralizing the reaction mixture with a 10 % HCl solution. The reaction was completed within 2 hours. Then water is distilled off from the reaction mixture under reduced pressure and 500 ml of ethanol was added to the residue followed by heating. After filtering off the insoluble salt, the filtrate was concentrated. Thus 26 g of the aimed product was obtained in the form of a white or pale yellow solid.

The molecular weight and spectral data of the obtained product were as follows.
Decomposing Point: 150 °C
Molecular .Weight.: 1241.6.
Spectral data:
   ¹H-NMR (MEOH-d₄); (ppm)
   0.1 (Si-CH₃, S, 12H), 0.55 (Si-CH₂-, t 8.7 Hz, 8H), 1.53 (Si-CH₂-CH₂-, m, 8H), 3.07 (Si-CH₂-CH₂-CH₂-O-, t 6 Hz, 8H), 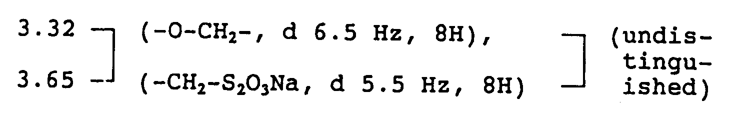 4.75 (>CH-OH, m, 4H)
   ¹³C-NMR (MeOH-d₄): (ppm)
   1.1 (Si-CH₃), 15.0 (Si-CH₂-), 25.2 (-CH₂-), 40.2 (-CH₂-S₂O₃Na), 71.8 (>CHOH), 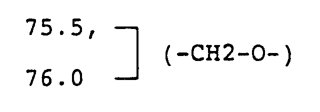
IR (KBr) ν max:
   3446 cm⁻¹ (-OH), 1038 cm⁻¹ (Si-O)

### EXAMPLE 8

Two-pack system compositions (Products of the Invention 5-1 to 5-8 and comparative Products 5-1 to 5-4) of the formulations as specified in Tables 7 and 8 were prepared. About 20 g of each composition was applied to about 20 g of the permed black hair of a Japanese subject by the method as specified in Table 7 or 8.

Tables 7 and 8 show the effects thus achieved.

### EXAMPLE 9

Two-pack system compositions (Products of the Invention 5-9 to 5-15 and Comparative Products 5-5 and 5-8) of the formulations as specified in Tables 9 and 10 were prepared. About 20 g of each composition was applied to about 20 g of the permed black hair of a Japanese subject by the method as specified in Table 9 or 10.

Tables 9 and 10 show the effects thus achieved.

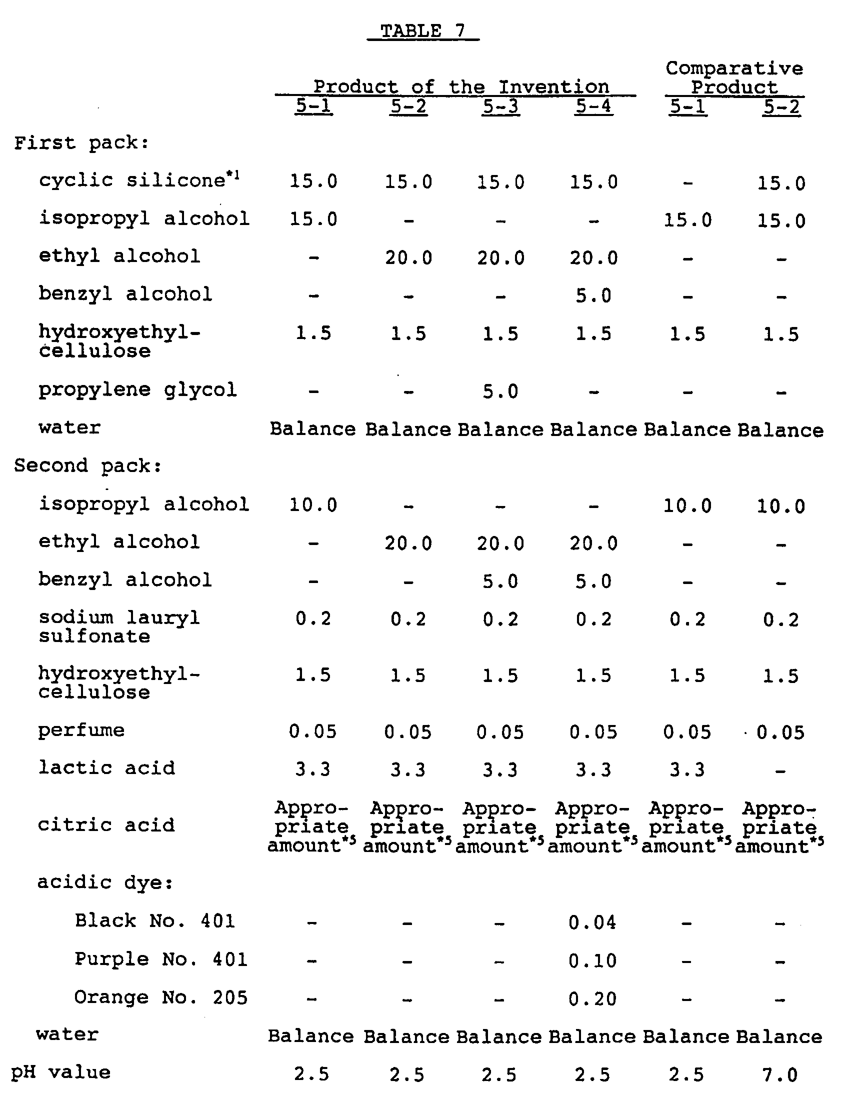

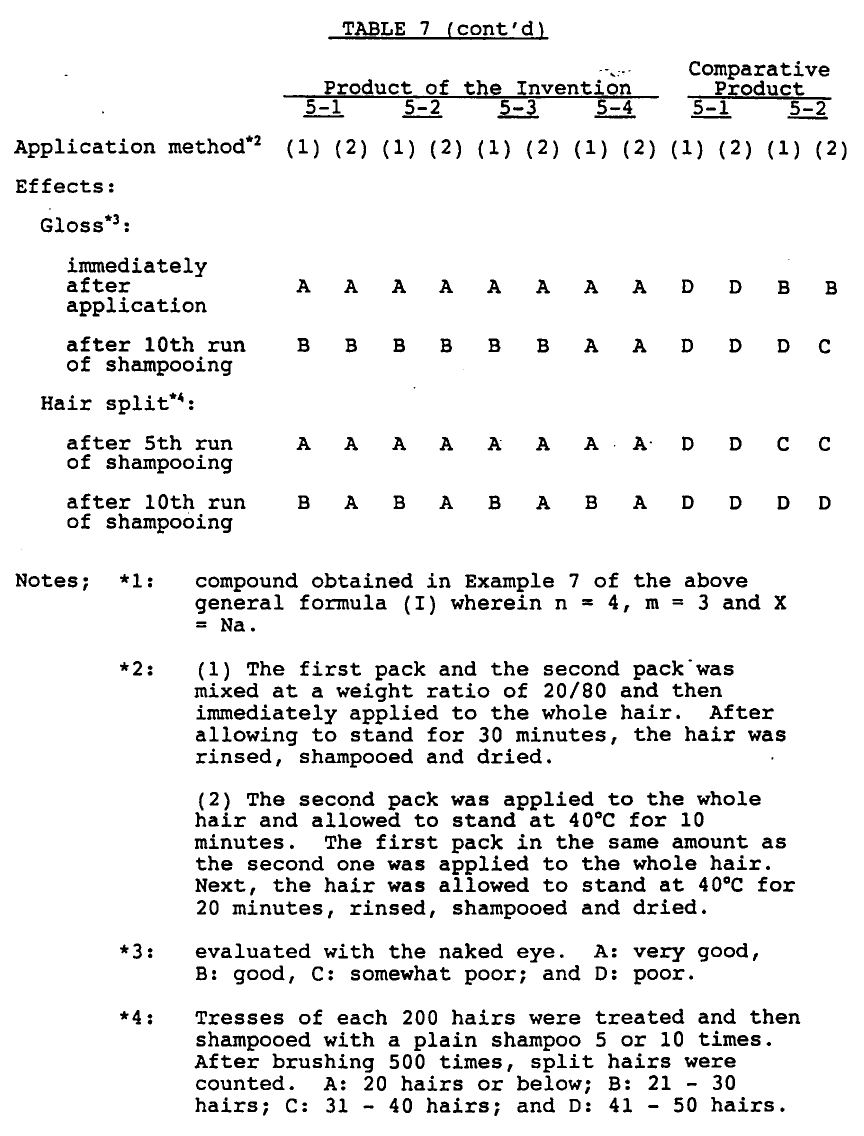

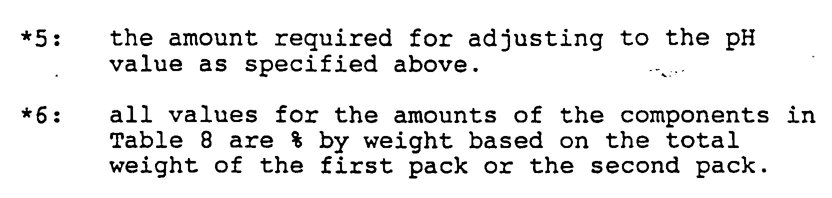

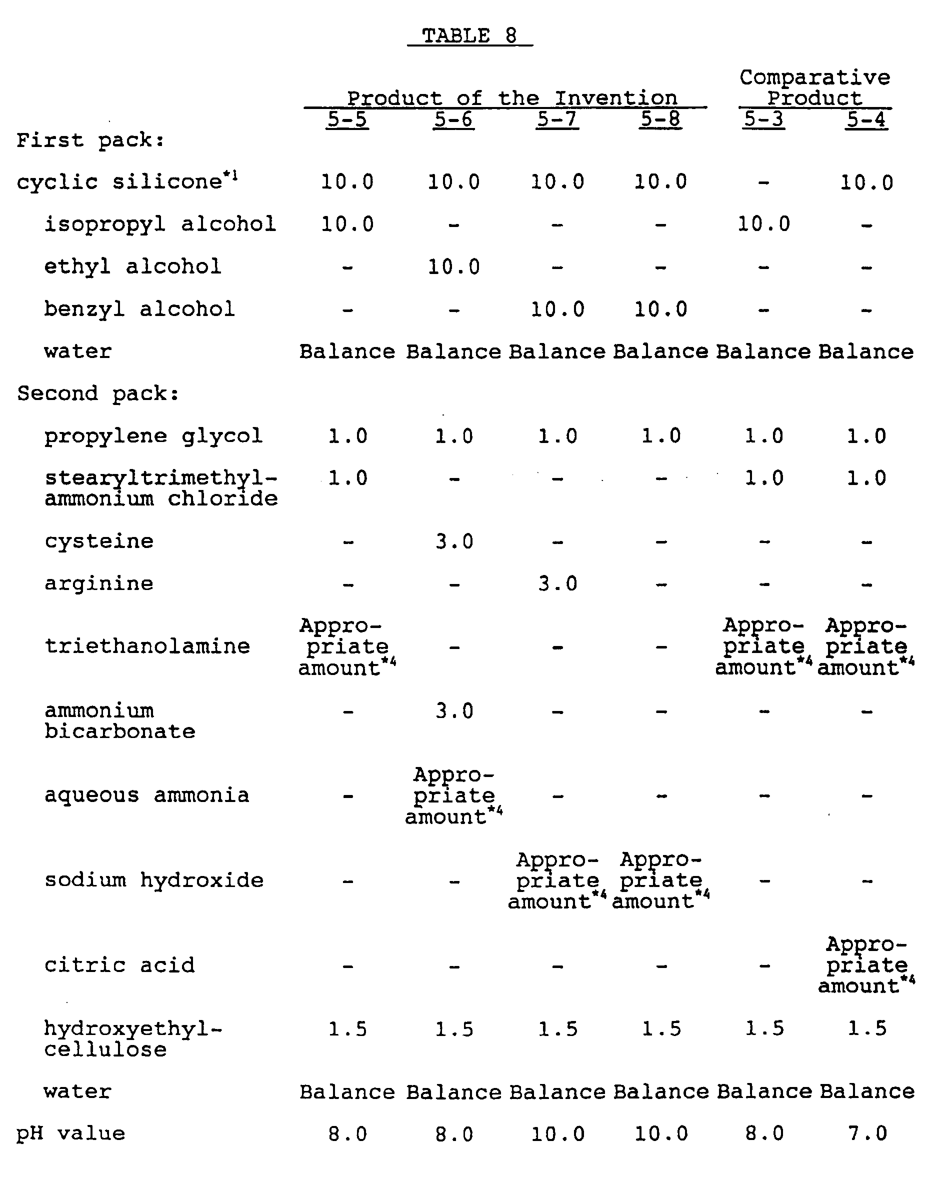

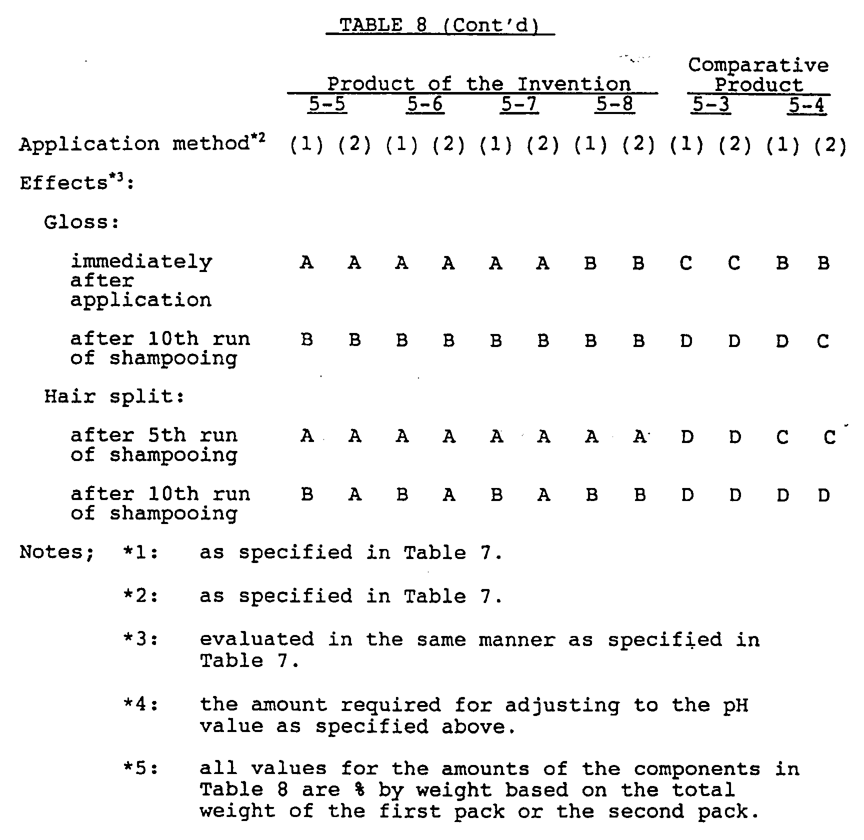

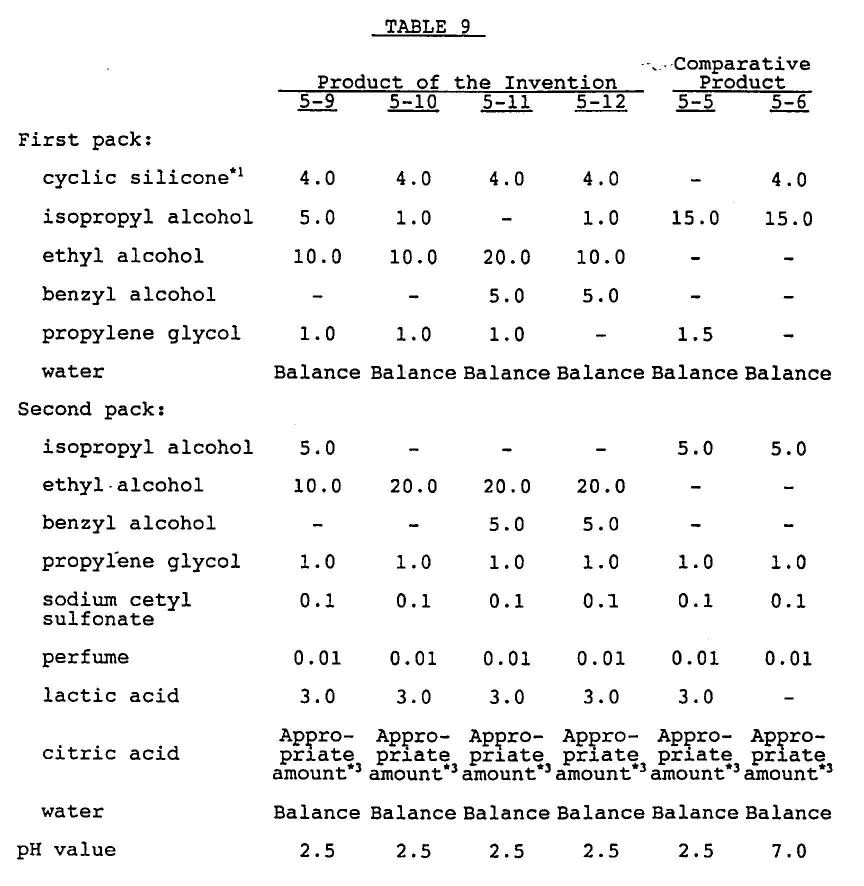

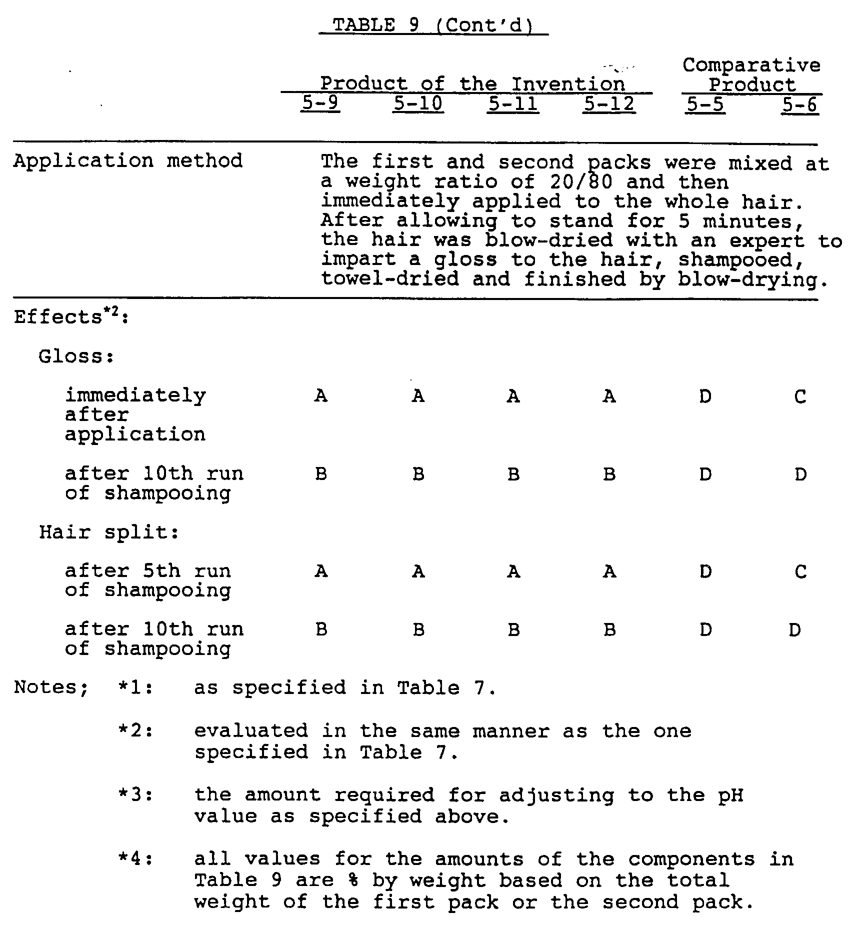

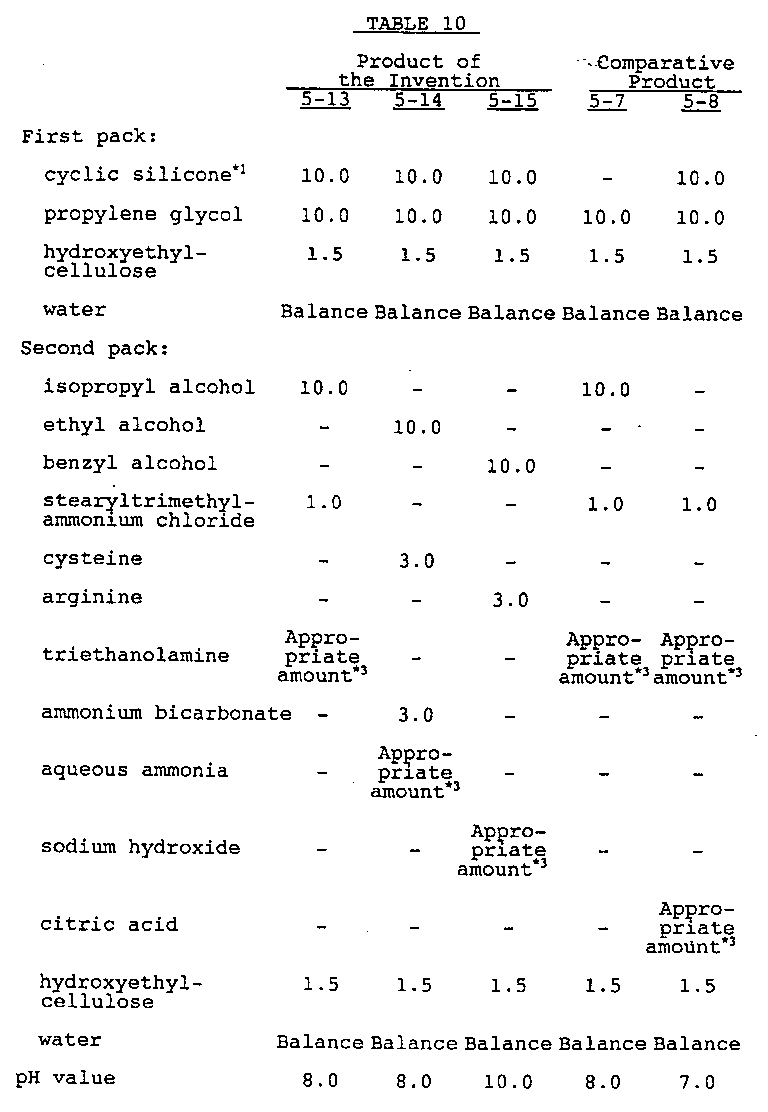

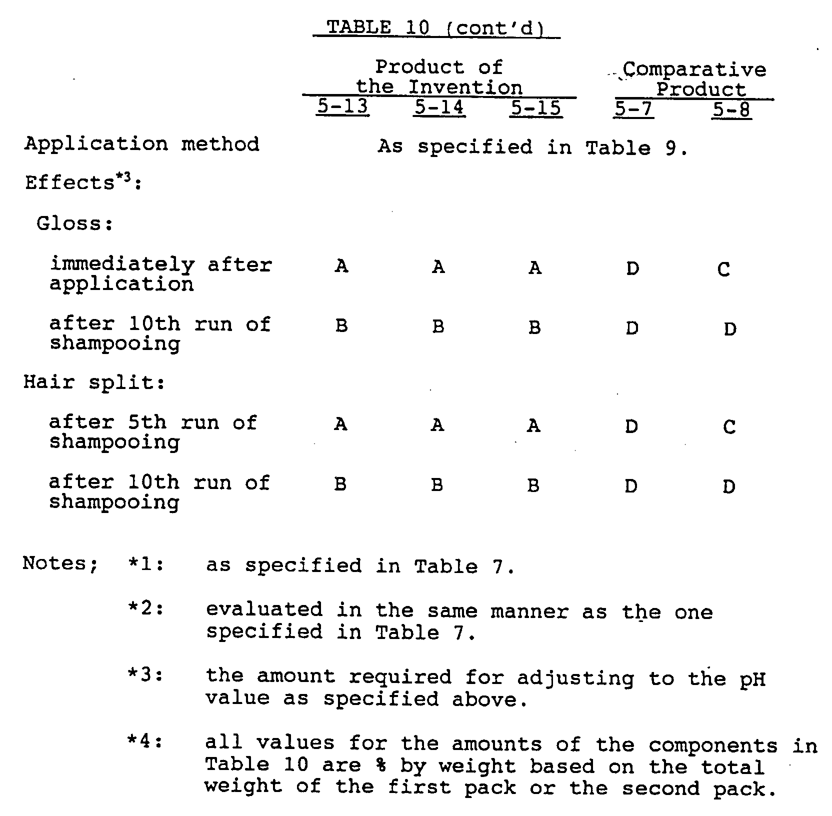

### EXAMPLE 10

Two-pack system compositions (Products of the Invention 6-1 to 6-3 and Comparative Products 6-1 and 6-2) of the formulations as specified in Table 11 were prepared. About 20 g of each composition was applied to about 20 g of the permed black hair of a Japanese subject by the method as specified in Table 11.

Table 11 shows the effects thus achieved.

### EXAMPLE 11

Two-pack system compositions (Products of the Invention 6-4 to 6-6 and Comparative Products 6-3 and 6-4) of the formulations as specified in Table 12 were prepared. About 20 g of each composition was applied to about 20 g of the permed black hair of a Japanese subject by the method as specified in Table 12.

Table 12 shows the effects thus achieved.

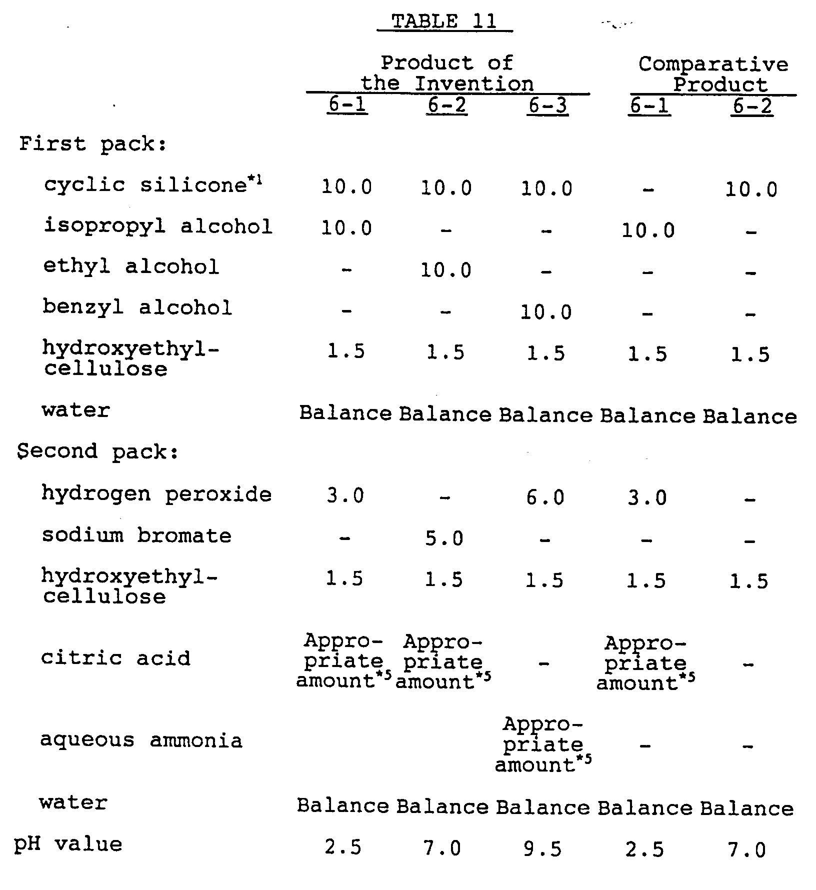

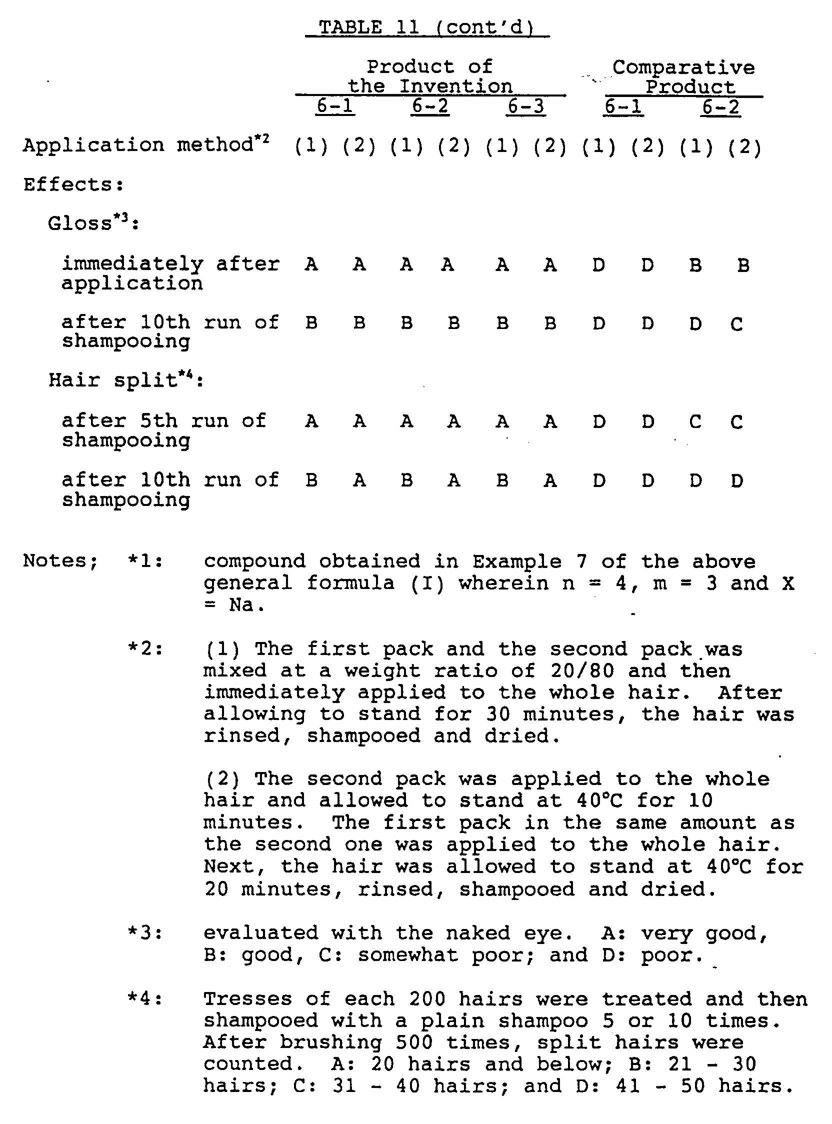

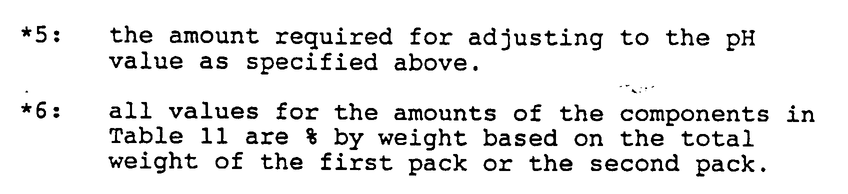

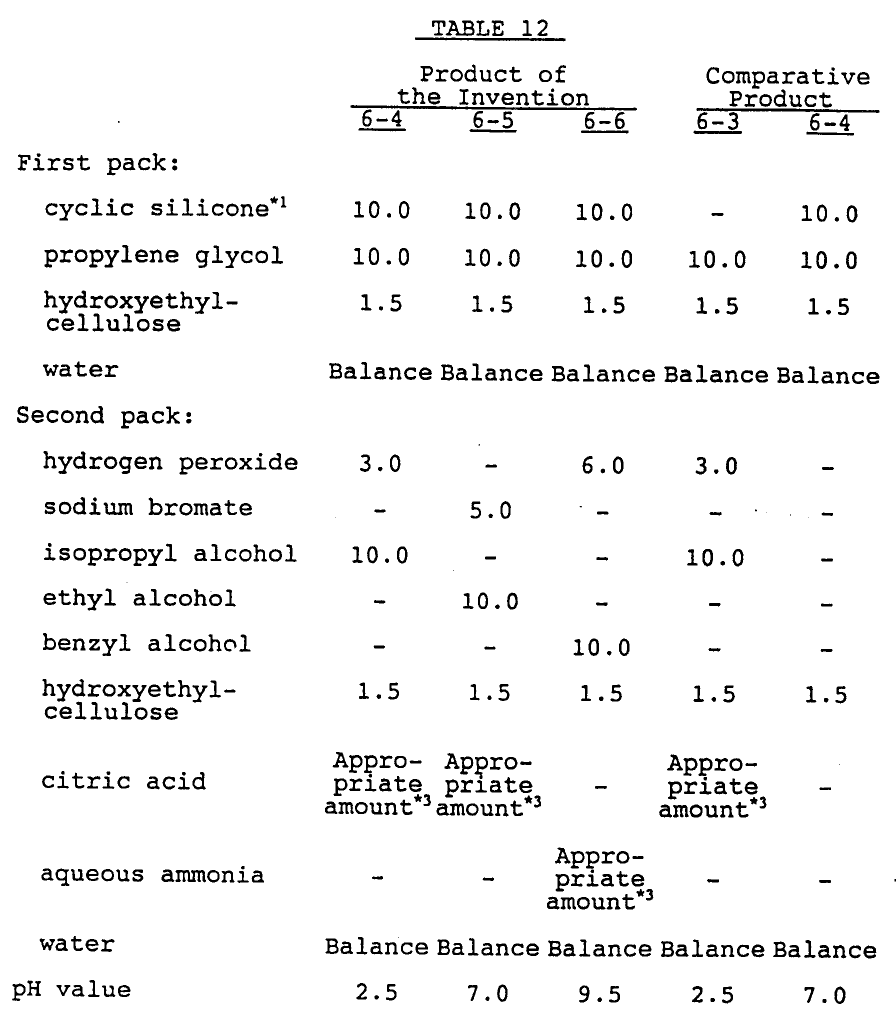

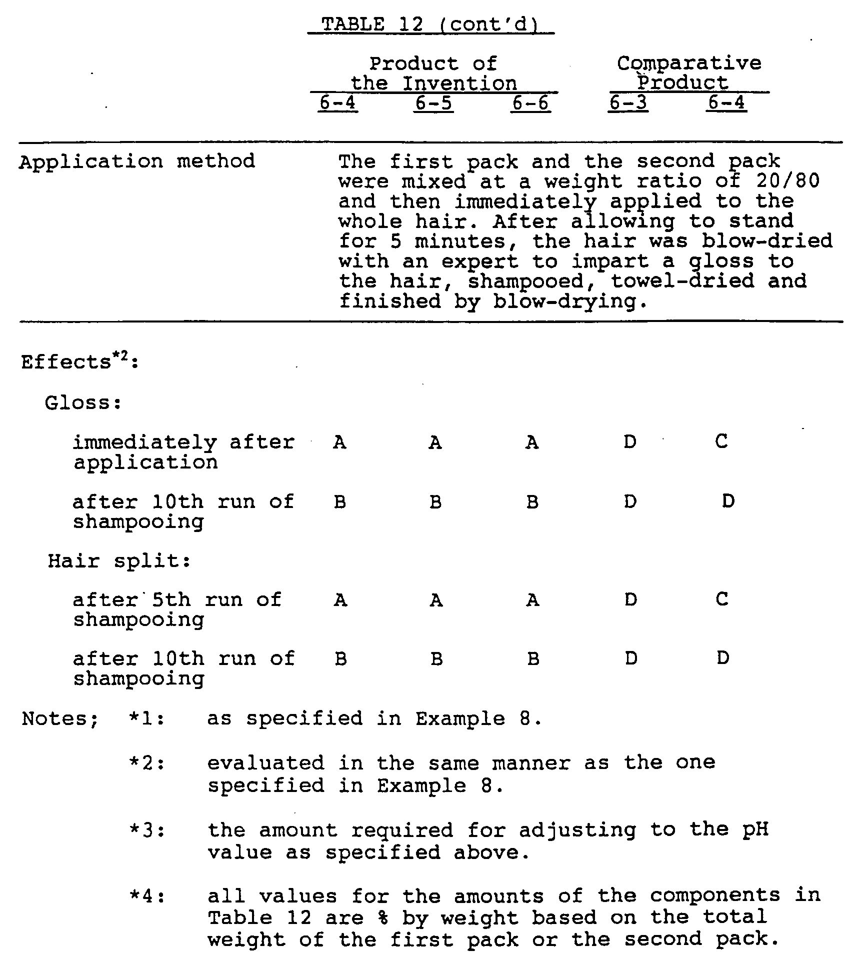

### EXAMPLE 12

Two-pack system compositions (Products of the Invention 7-1 to 7-3 and Comparative Products 7-1 and 7-2) of the formulations as specified in Table 13 were prepared. About 20 g of each composition was applied to about 20 g of the permed black hair of a Japanese subject by the method as specified in Table 13.

Table 13 shows the effects thus achieved.

### EXAMPLE 13

Two-pack system compositions (Products of the Invention 7-4 to 7-6 and Comparative Products 7-3 and 7-4) of the formulations as specified in Table 14 were prepared. About 20 g of each composition was applied to about 20 g of the permed black hair of a Japanese subject by the method as specified in Table 14.

Table 14 shows the effects thus achieved.

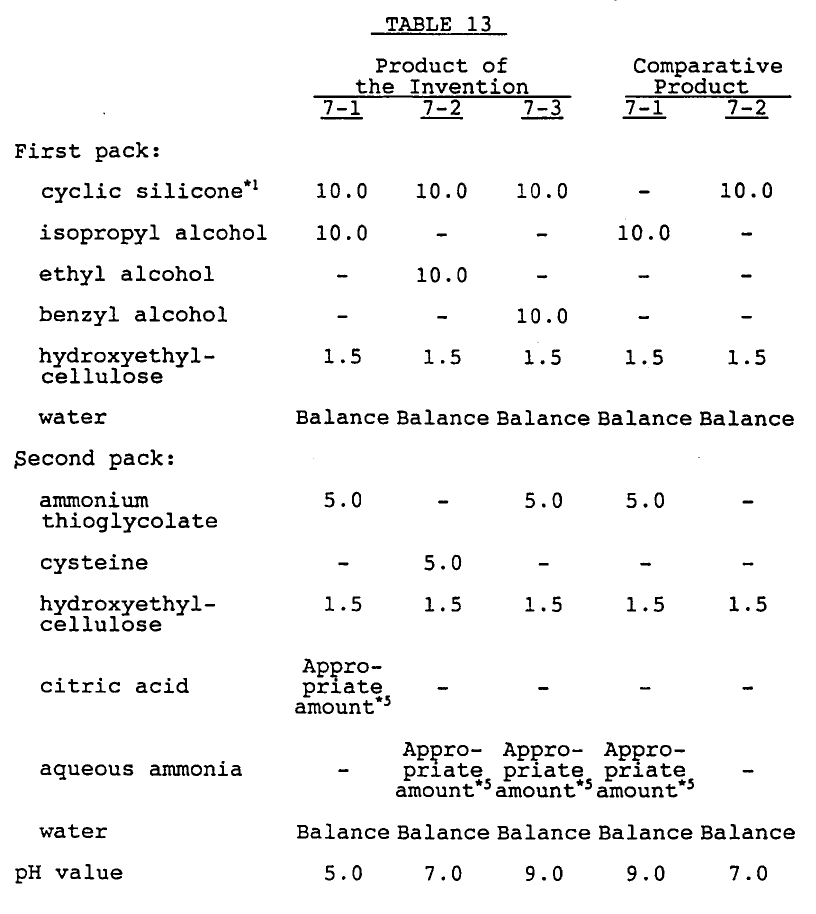

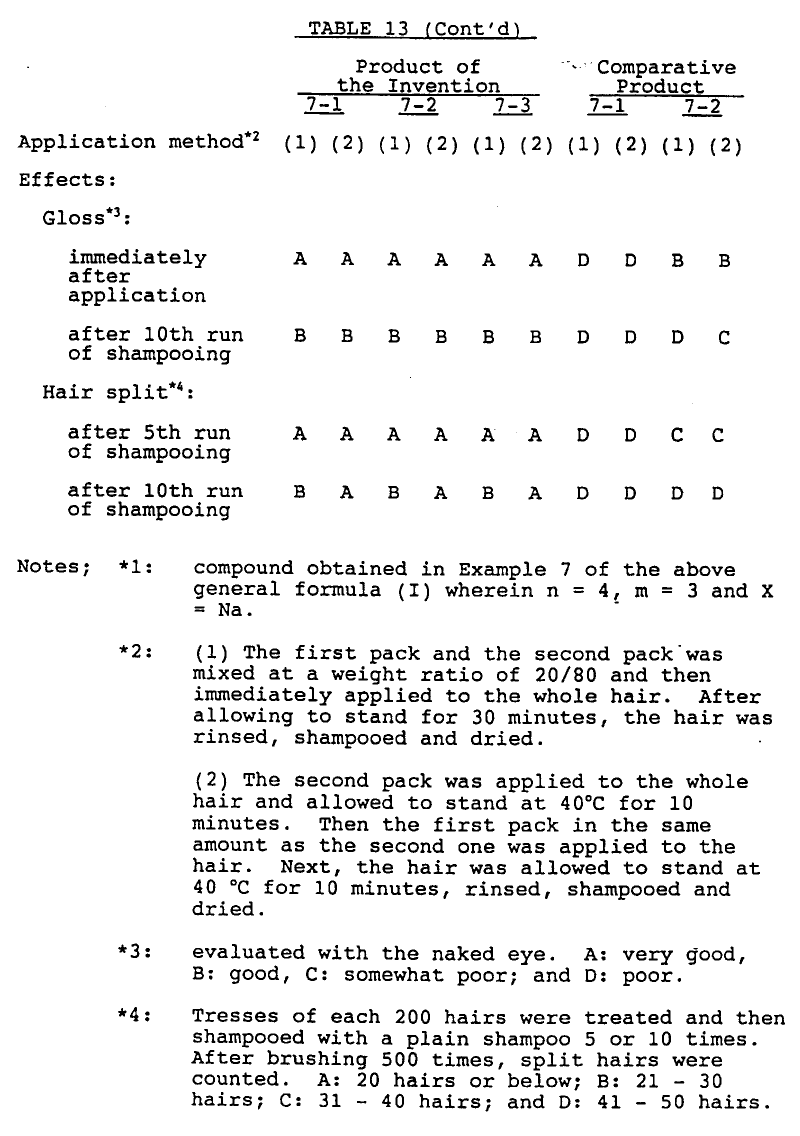

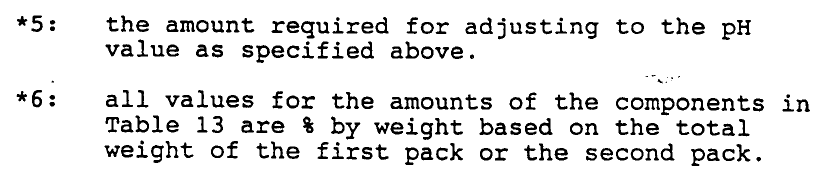

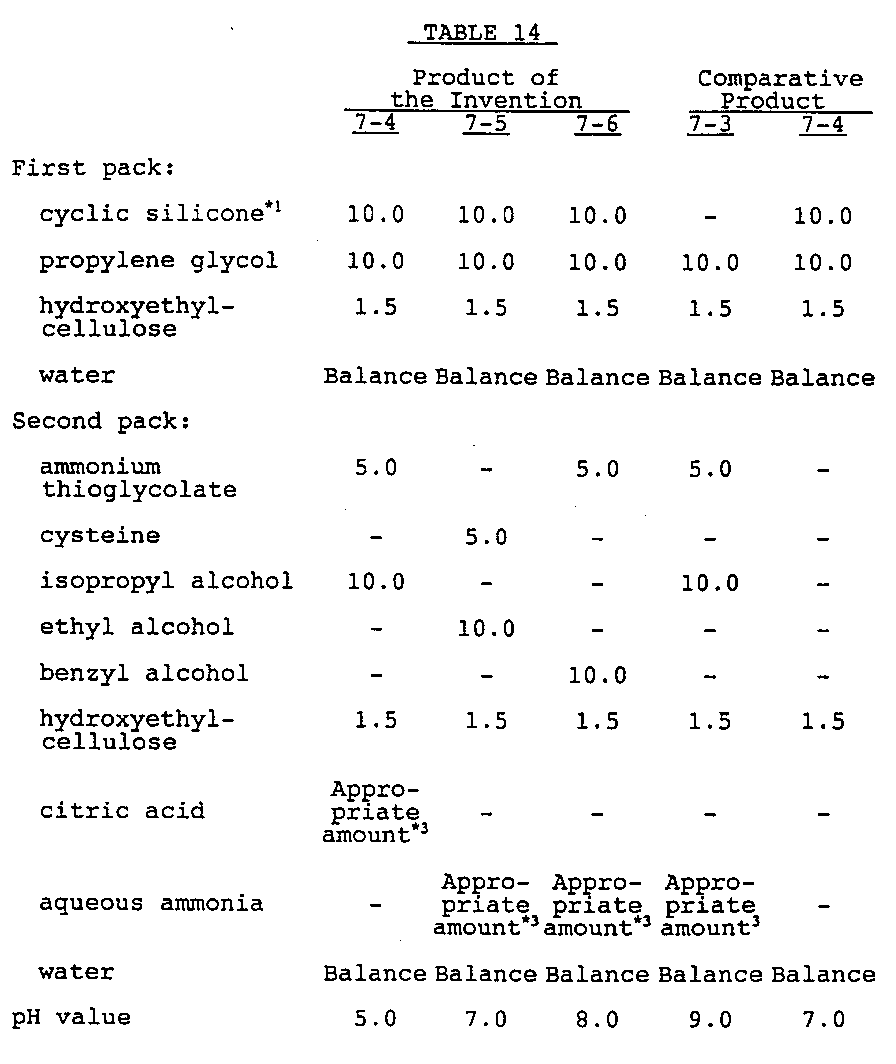

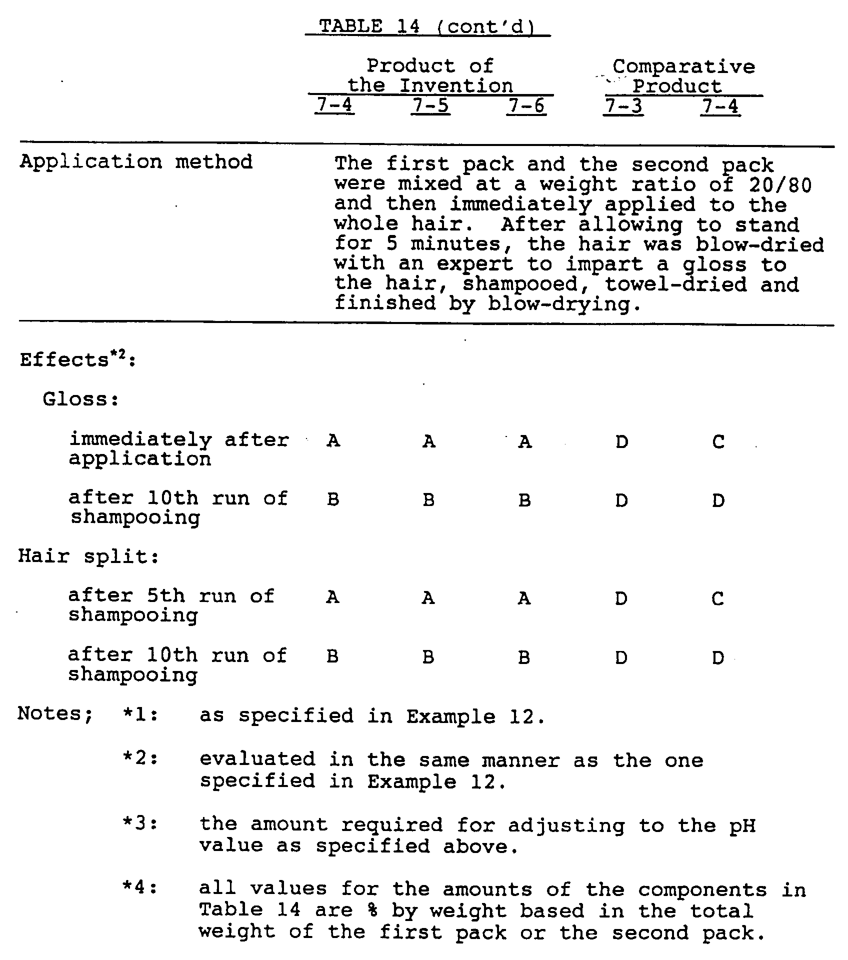

According to the hair treatment composition of the present invention, the conditioning components adsorb on the surface of the hair and then penetrate into the hair. As a result, a coating film which is sustained for a long period of time is formed on the surface of the hair. Thus an effect of preventing hair from causing a split hair and a gloss can be imparted to the hair. When heating is conducted in a daily hair-care process such as blowing or in a beauty salon, furthermore, a coating film which can be sustained for a longer period of time can be formed on the damaged part of the hair. Thus the effect of preventing hair from causing a split hair and effect of improving hair gloss can be further enhanced.

The novel cyclic Bunte salt-modified silicone of the present invention is useful as, in particular, a conditioning component of a hair treatment composition. The hair treatment composition of the present invention comprising silicone can make the hair glossy. Furthermore, the cyclic Bunte salt-modified silicone of the present invention is also useful as a surface treatment agent for textile, fiber and paper.

## Claims

1. A hair treatment composition which comprises:
(a) one or more compounds represented by the following general formula (I) or (II); and
(b) one or more compounds represented by the following general formula (III) or (IV);
and the pH value thereof is adjusted to 2 to 4 or 8 to 11 at the use: 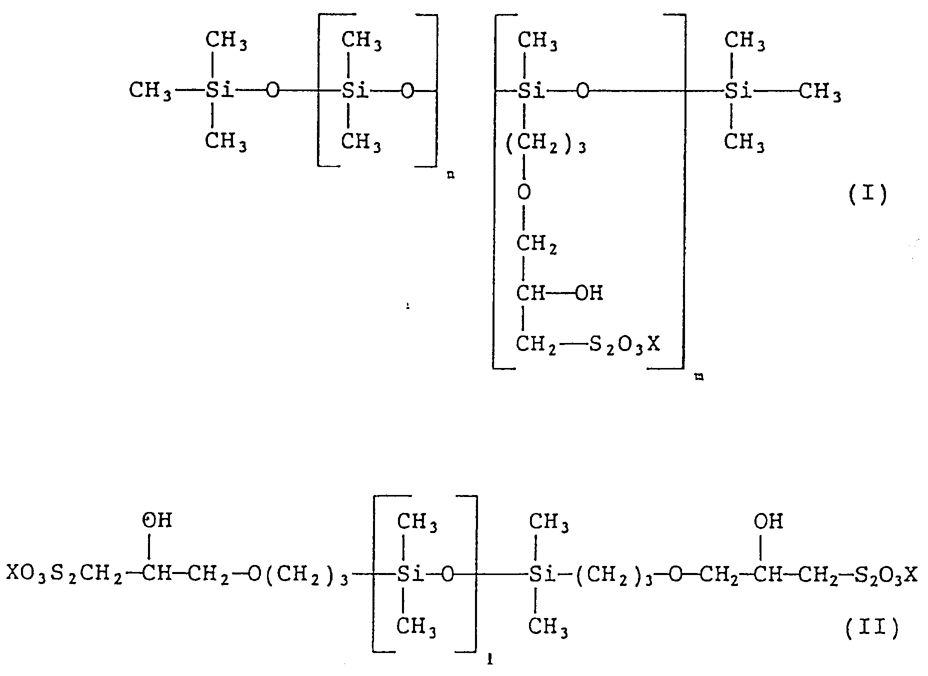
wherein X represents an alkali metal, an alkaline earth metal, an ammonium, a primary ammonium, a secondary ammonium, a tertiary ammonium or a quaternary ammonium; n is an integer of from 1 to 20;
m is an integer of from 1 to 20, provided that n/m > 1; and ℓ is an integer of from 1 to 20; 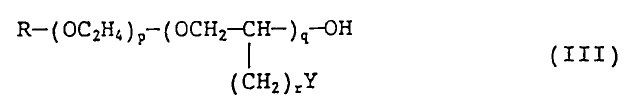
wherein R represents a hydrogen atom or an 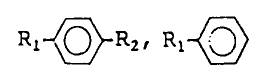 or a straight-chain or branched alkyl group having 1 to 4 carbon atoms, in which R₁ represents a hydrogen atom, a methyl group or a methoxy group; and
R₂ represents a -CH₂-, -C(CH₃)₂-, -CH₂-CH(CH₃)- or -CH=CH-CH₂- group; Y represents a hydrogen atom or a hydroxyl group; and p, q and r are each an integer of from 0 to 5, provided that at least one of p and q is 0j and provided that in the compound of formula (III) p and q may not be 0 at the same time, when R is hydrogen; 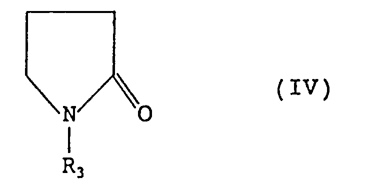
wherein R₃ represents a straight-chain or branched alkyl group having 8 to 18 carbon atoms.

2. A hair treatment composition of claim 1, wherein said component (a) amounts from 0.01 to 50 % by weight and said component (b) amounts from 0.1 to 50 % by weight.

3. A hair treatment composition of claim 1, wherein said component (a) is a mixture of a compound of the general formula (I) and a compound of the general formula (II) and a weight ratio of the compound of the general formula (I) to the compound of the general formula (II) (the compound of the general formula (I)/the compound of the general formula (II)) ranges from 1/10 to 10/1.

4. A hair treatment composition of claim 1, wherein a weight ratio of said component (a) to said component (b) ((a)/(b)) ranges from 1/20 to 20/1.

5. A hair treatment composition of any of the claims 1 to 4, wherein additionally a component (c) is comprised being represented by one or more compounds selected from the group consisting of ammonia, amino compounds, amines and quaternary ammonium salts; and wherein the pH value thereof is adjusted to 8 to 11 at the use.

6. A hair treatment composition of claim 5, wherein said hair treatment composition is provided in the form of a two-pack type composition consisting of:
the first pack comprising said Component (a), or said Component (a) and said Component (b); and
the second pack comprising said Component (b) and Component (c), or said Component (c).

7. A hair treatment composition of claim 5, wherein said component (c) amounts from 0.1 to 10% by weight.

8. A hair treatment composition which comprises:
(a) one or more compounds represented by the following general formula (I) or (II);
(b) one or more compounds represented by the following general formula (III) or (IV); and
(d) one or more oxidizing agents: 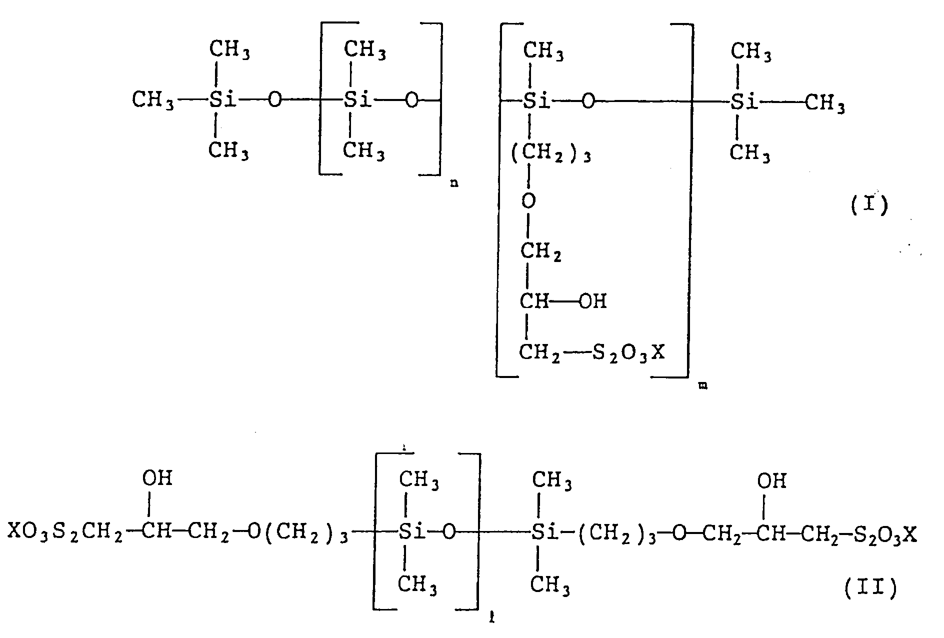
wherein X represents an alkali metal, an alkaline earth metal, an ammonium, a primary ammonium, a secondary ammonium, a tertiary ammonium or a quaternary ammonium; n is an integer of from 1 to 20; m is an integer of from 1 to 20, provided that n/m > 1; and ℓ is an integer of from 1 to 20; 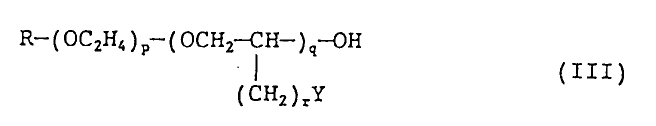
wherein R represents a hydrogen atom or an 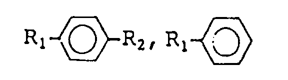 or a straight-chain or branched alkyl group having 1 to 4 carbon atoms, in which R₁ represents a hydrogen atom, a methyl group or a methoxy group, and R₂ represents a -CH₂-, -C(CH₃)₂-, -CH₂-CH(CH₃)- or -CH=CH-CH₂- group; Y represents a hydrogen atom or a hydroxyl group; and p, q and
r are each an integer of from 0 to 5, provided that at least one of p and q is 0j and provided that in the compound of formula (III) p and q may not be 0 at the same time, when R is hydrogen; 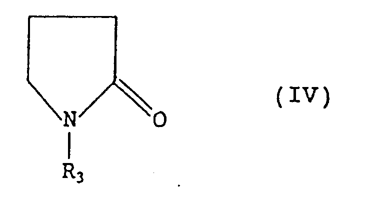
wherein R₃ represents a straight-chain or branched alkyl group having 8 to 18 carbon atoms.

9. A hair treatment composition of claim 8 wherein said hair treatment composition is provided in the form of a two-pack type composition consisting of: the first pack comprising said Component (a), or said Component (a) and said Component (b); and the second pack comprising said Component (b) and said Component (d), or said Component (d).

10. A hair treatment composition of claim 8, wherein said component (a) amounts from 0.01 to 50 % by weight, said component (b) amounts from 0.1 to 50 % by weight and said component (d) amounts from 0.05 to 30 % by weight.

11. A hair treatment composition of claim 8, wherein said component (a) is a mixture of a compound of the general formula (I) and a compound of the general formula (II) and a weight ratio of the compound of the general formula (I) to the compound of the general formula (II) (the compound of the general formula (I)/the compound of the general formula (II)) ranges from 1/10 to 10/1.

12. A hair treatment composition of claim 8, wherein a weight ratio of said component (a) to said component (b) ((a)/(b)) ranges from 1/20 to 20/1.

13. A cyclic Bunte salt-modified silicone represented by the following general formula (V): 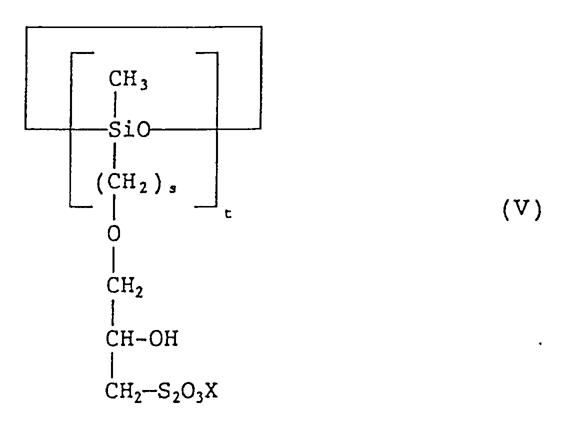
wherein X represents an alkali metal, an alkaline earth metal, an ammonium, a primary ammonium, a secondary ammonium, a tertiary ammonium or a quaternary ammonium; t is an integer of from 4 to 10; and s is an integer of from 3 to 11.

14. A hair treatment composition which comprises one or more cyclic Bunte salt-modified silicones as defined in claim 13, and the pH value thereof is adjusted to 2 to 4 or 8 to 11 at the use.

15. A hair treatment composition of claim 14, wherein said one or more cyclic Bunte salt-modified silicones amount from 0.01 to 50 % by weight.

16. A hair treatment composition which comprises:
(e) one or more cyclic Bunte salt-modified scones as defined in claim 13; and
(d) one or more oxidizing agents.

17. A hair treatment composition of claim 16, wherein said hair treatment composition is provided in the form of a two-pack type composition consisting of:
the first pack comprising said Component (e); and
the second pack comprising said Component (d).

18. A hair treatment composition of claim 16, wherein said component (e) amounts from 0.01 to 50 % by weight and said component (d) amounts from 0.05 to 30 % by weight.

19. A hair treatment composition which comprises:
(e) one or more cyclic Bunte salt-modified silicones as defined in claim 13; and
(f) one or more reducing agents.

20. A hair treatment composition of claim 19, wherein said hair treatment composition is provided in the form of a two-pack type composition consisting of:
the first pack comprising said Component (e); and
the second pack comprising said Component (f).

21. A hair treatment composition of claim 19, wherein said component (e) amounts from 0.01 to 50 % by weight and said component (f) amounts from 0.1 to 20 % by weight.

## Patentansprüche

1. Haarbehandlungs-Zusammensetzung, die folgendes umfasst:
(a) eine oder mehrere Verbindungen der folgenden allgemeinen Formel (I) oder (II); und
(b) eine oder mehrere Verbindungen der folgenden allgemeinen Formel (III) oder (IV);
und deren pH-Wert zum Zeitpunkt der Anwendung auf 2 bis 4 oder 8 bis 11 eingestellt ist: 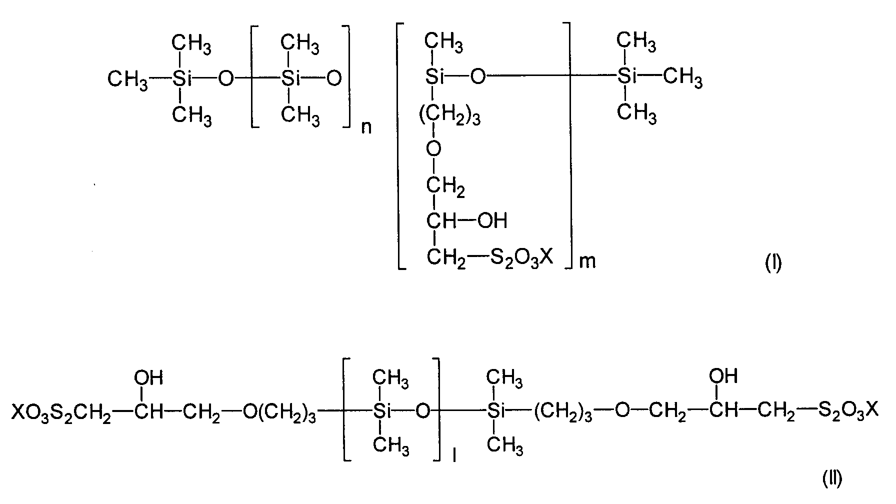
worin X ein Alkalimetall, ein Erdalkalimetall, ein Ammonium, ein primäres Ammonium, ein sekundäres Ammonium, ein tertiäres Ammonium oder ein quaternäres Ammonium darstellt; n ist eine ganze Zahl von 1 bis 20; m ist eine ganze Zahl von 1 bis 20,
vorausgesetzt, dass n/m > 1; und ℓ ist eine ganze Zahl von 1 bis 20; 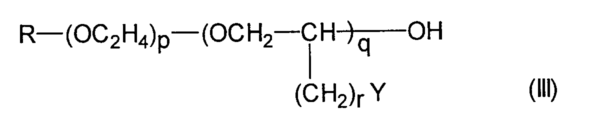
worin R ein Wasserstoffatom oder, 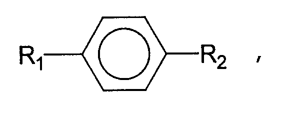 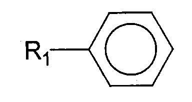 oder eine unverzweigte oder verzweigte Alkylgruppe mit 1 bis 4 Kohlenstoffatomen repräsentiert, worin R₁ ein Wasserstoffatom, eine Methylgruppe oder eine Methoxygruppe repräsentiert, und R₂ repräsentiert eine -CH₂-, -C(CH₃)₂-, -CH₂-CH(CH₃)- oder -CH=CH-CH₂-Gruppe; Y repräsentiert ein Wasserstoffatom oder eine Hydroxylgruppe; und p, q und r sind jeweils ganze Zahlen von 0 bis 5, vorausgesetzt, dass mindestens eines von p und q 0 ist; und vorausgesetzt, dass in der Verbindung der Formel (III) p und q nicht gleichzeitig 0 sind, wenn R Wasserstoff ist; 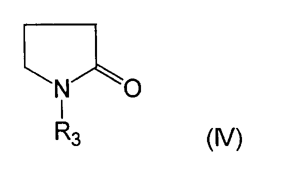 worin R₃ eine unverzweigte oder verzweigte Alkylgruppe mit 8 bis 18 Kohlenstoffatomen repräsentiert.

2. Haarbehandlungs-Zusammensetzung gemäss Anspruch 1, dadurch **gekennzeichnet,** dass die Komponente (a) in einer Menge von 0,01 bis 50 Gew.% und die Komponente (b) in einer Menge von 0,1 bis 50 Gew.% enthalten ist.

3. Haarbehandlungs-Zusammensetzung gemäss Anspruch 1, dadurch **gekennzeichnet,** dass Komponente (a) eine Mischung einer Verbindung der allgemeinen Formel (I) und einer Verbindung der allgemeinen Formel (II) darstellt, und das Gewichtsverhältnis der Verbindung der allgemeinen Formel (I) zur Verbindung der allgemeinen Formel (II) [Verbindung der allgemeinen Formel (I)/Verbindung der allgemeinen Formel (II)] im Bereich von 1:10 bis 10:1 liegt.

4. Haarbehandlungs-Zusammensetzung gemäss Anspruch 1, dadurch **gekennzeichnet,** dass das Gewichtsverhältnis der Komponente (a) zur Komponente (b) [(a)/(b)] im Bereich von 1:20 bis 20:1 liegt.

5. Haarbehandlungs-Zusammensetzung gemäss mindestens einem der Ansprüche 1 bis 4, dadurch **gekennzeichnet,** dass zusätzlich eine Komponente (c) enthalten ist, die repräsentiert wird durch eine oder mehrere Verbindungen, ausgewählt aus der Gruppe, bestehend aus Ammoniak, Aminoverbindungen, Aminen und quaternären Ammoniumsalzen, und worin der pH-Wert zum Zeitpunkt der Verwendung auf 8 bis 11 eingestellt ist.

6. Haarbehandlungs-Zusammensetzung gemäss Anspruch 5, dadurch **gekennzeichnet,** dass die Haarbehandlungs-Zusammensetzung bereitgestellt wird in Form einer Zweipackungstyp-Zusammensetzung, bestehend aus:
einer ersten Packung, die die Komponente (a) oder die Komponente (a) und die Komponente (b) umfasst; und
einer zweiten Packung, die die Komponente (b) und Komponente (c), oder Komponente (c) umfasst.

7. Haarbehandlungs-Zusammensetzung gemäss Anspruch 5, dadurch **gekennzeichnet,** dass die Komponente (c) in einer Menge von 0,1 bis 10 Gew.% enthalten ist.

8. Haarbehandlungs-Zusammensetzung, die folgendes umfasst:
(a) eine oder mehrere Verbindungen der folgenden allgemeinen Formel (I) oder (II);
(b) eine oder mehrere Verbindungen der folgenden allgemeinen Formel (III) oder (IV); und
(d) ein oder mehrere Oxidationsmittel: 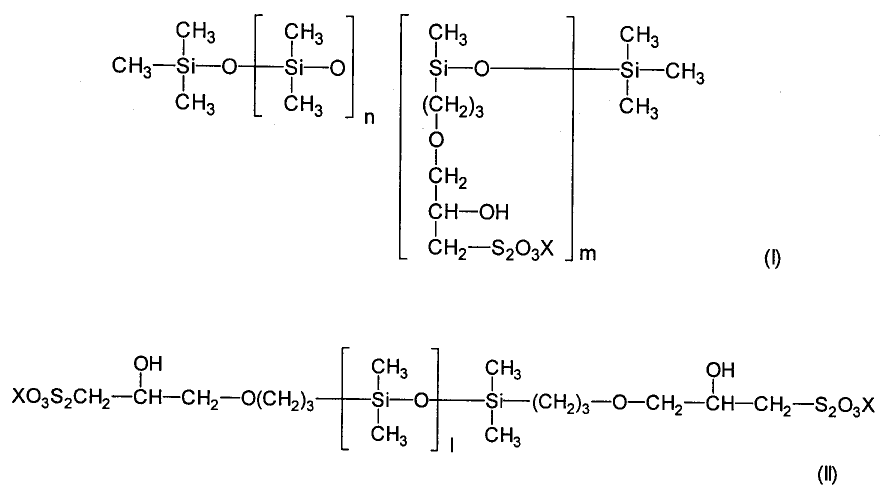
worin X ein Alkalimetall, ein Erdalkalimetall, ein Ammonium, ein primäres Ammonium, ein sekundäres Ammonium, ein tertiäres Ammonium oder ein quaternäres Ammonium darstellt; n ist eine ganze Zahl von 1 bis 20; m ist eine ganze Zahl von 1 bis 20, vorausgesetzt, dass n/m > 1; und ℓ ist eine ganze Zahl von 1 bis 20; 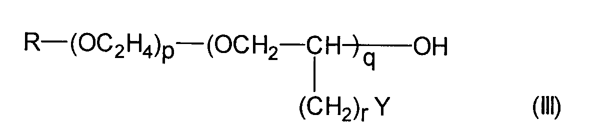
worin R ein Wasserstoffatom oder, 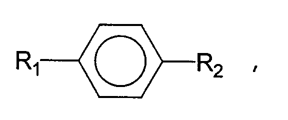 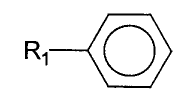 oder eine unverzweigte oder verzweigte Alkylgruppe mit 1 bis 4 Kohlenstoffatomen repräsentiert, worin R₁ ein Wasserstoffatom, eine Methylgruppe oder eine Methoxygruppe repräsentiert, und R₂ repräsentiert eine -CH₂-, -C(CH₃)₂-, -CH₂-CH(CH₃)- oder -CH=CH-CH₂-Gruppe; Y repräsentiert ein Wasserstoffatom oder eine Hydroxylgruppe; und p, q und r sind jeweils ganze Zahlen von 0 bis 5, vorausgesetzt, dass mindestens eines von p und q 0 ist; und vorausgesetzt, dass in der Verbindung der Formel (III) p und q nicht gleichzeitig 0 sind, wenn R Wasserstoff ist; 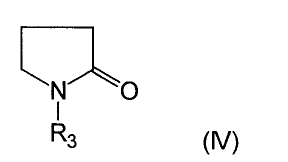
worin R₃ eine unverzweigte oder verzweigte Alkylgruppe mit 8 bis 18 Kohlenstoffatomen repräsentiert.

9. Haarbehandlungs-Zusammensetzung gemäss Anspruch 8, dadurch **gekennzeichnet,** dass die Haarbehandlungs-Zusammensetzung bereitgestellt wird in Form einer Zweipackungstyp-Zusammensetzung, bestehend aus:
einer ersten Packung, die die Komponente (a) oder die Komponente (a) und die Komponente (b) umfasst; und
der zweiten Packung, die die Komponente (b) und die Komponente (d), oder die Komponente (d) umfasst.

10. Haarbehandlungs-Zusammensetzung gemäss Anspruch 8, dadurch **gekennzeichnet,** dass die Komponente (a) in einer Menge von 0,01 bis 50 Gew.%, die Komponente (b) in einer Menge von 0,1 bis 50 Gew.%, und die Komponente (d) in einer Menge von 0,05 bis 30 Gew.% enthalten ist.

11. Haarbehandlungs-Zusammensetzung gemäss Anspruch 8, dadurch **gekennzeichnet,** dass die Komponente (a) eine Mischung einer Verbindung der allgemeinen Formel (I) und einer Verbindung der allgemeinen Formel (II) darstellt, und das Gewichtsverhältnis der Verbindung der allgemeinen Formel (I) zur Verbindung der allgemeinen Formel (II) [Verbindung der allgemeinen Formel (I)/Verbindung der allgemeinen Formel (II)] im Bereich von 1:10 bis 10:1 liegt.

12. Haarbehandlungs-Zusammensetzung gemäss Anspruch 8, dadurch **gekennzeichnet,** dass das Gewichtsverhältnis der Komponente (a) zur Komponente (b) [(a)/(b)] im Bereich von 1:20 bis 20:1 liegt.

13. Cyclisches Buntesalz-modifiziertes Silicon der folgenden allgemeinen Formel (V): 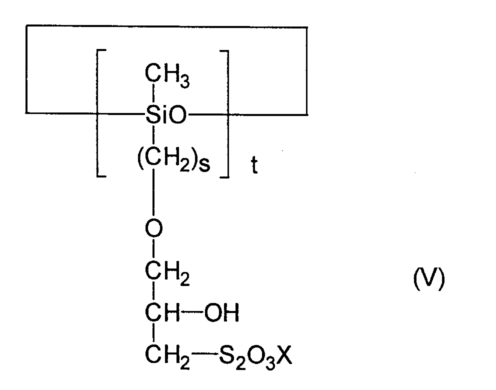
worin X ein Alkalimetall, ein Erdalkalimetall, ein Ammonium, ein primäres Ammonium, ein sekundäres Ammonium, ein tertiäres Ammonium oder ein quaternäres Ammonium darstellt; t ist eine ganze Zahl von 4 bis 10; und s ist eine ganze Zahl von 3 bis 11.

14. Haarbehandlungs-Zusammensetzung, die ein oder mehrere cyclische Buntesalz-modifizierte Silicone, wie in Anspruch 13 definiert, enthält, und deren pH-Wert zum Zeitpunkt der Anwendung auf 2 bis 4 oder 8 bis 11 eingestellt ist.

15. Haarbehandlungs-Zusammensetzung gemäss Anspruch 14, dadurch **gekennzeichnet,** dass ein oder mehrere cyclische Buntesalz-modifizierte Silicone in einer Menge von 0,01 bis 50 Gew.% enthalten sind.

16. Haarbehandlungs-Zusammensetzung, die
(e) ein oder mehrere cyclische Buntesalzmodifizierte Silicone, wie in Anspruch 13 definiert; und
(d) ein oder mehrere Oxidationsmittel enthält.

17. Haarbehandlungs-Zusammensetzung gemäss Anspruch 16, dadurch **gekennzeichnet,** dass die Haarbehandlungs-Zusammensetzung bereitgestellt wird in Form einer Zweipackungstyp-Zusammensetzung, bestehend aus:
einer ersten Packung, die die Komponente (e) umfasst; und
einer zweiten Packung, die die Komponente (d) umfasst.

18. Haarbehandlungs-Zusammensetzung gemäss Anspruch 16, dadurch **gekennzeichnet,** dass die Komponente (e) in einer Menge von 0,01 bis 50 Gew.% und die Komponente (d) in einer Menge von 0,05 bis 30 Gew.% enthalten ist.

19. Haarbehandlungs-Zusammensetzung, die
(e) ein oder mehrere cyclische Buntesalzmodifizierte Silicone, wie in Anspruch 13 definiert; und
(f) ein oder mehrere Reduktionsmittel enthält.

20. Haarbehandlungs-Zusammensetzung gemäss Anspruch 19, dadurch **gekennzeichnet,** dass die Haarbehandlungs-Zusammensetzung bereitgestellt wird in Form einer Zweipackungstyp-Zusammensetzung, bestehend aus:
einer ersten Packung, die die Komponente (e) umfasst; und
einer zweiten Packung, die die Komponente (f) umfasst.

21. Haarbehandlungs-Zusammensetzung gemäss Anspruch 19, dadurch **gekennzeichnet,** dass die Komponente (e) in einer Menge von 0,01 bis 50 Gew.% und die Komponente (f) in einer Menge von 0,1 bis 20 Gew.% enthalten ist.

## Revendications

1. Une composition pour le traitement des cheveux qui comprend :
(a) un ou plusieurs composés représentés par la formule générale (I) ou (II) suivante ; et
(b) un ou plusieurs composés représentés par la formule générale suivante (III) ou (IV) ; et
dont la valeur de pH est ajustée à 2 à 4 ou à 8 à 11 au moment de l'emploi : 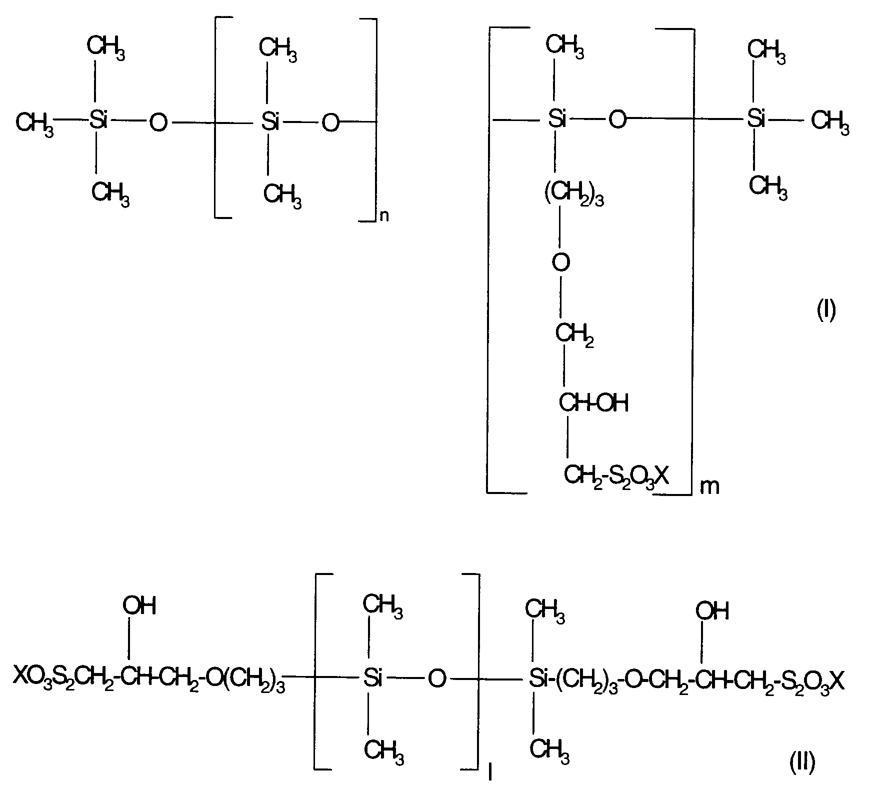
formule dans laquelle X représente un métal alcalin, un métal alcalino-terreux, un ammonium, un ammonium primaire, un ammonium secondaire, un ammonium tertiaire ou un ammonium quaternaire, n est un nombre entier de 1 à 20 ; m est un nombre entier de 1 à 20, à la condition que n/m>1 ; et l est un nombre entier de 1 à 20 ; 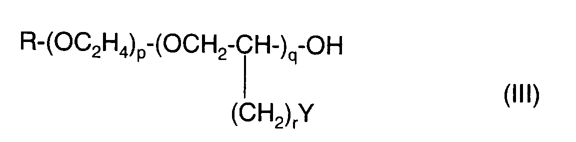
où R représente un atome d'hydrogène ou un groupe 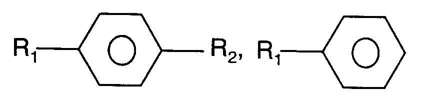 ou un groupe alkyle à chaîne linéaire ou ramifiée ayant de 1 à 4 atomes de carbone, dans lequel R₁ représente un atome d'hydrogène, un groupe méthyle ou un groupe méthoxy, et R₂ représente un groupe -CH₂-, -C(CH₃)₂-, -CH₂-CH(CH₃)- ou -CH=CH-CH₂-; Y représente un atome d'hydrogène ou un groupe hydroxyle ; et p, q et r désignent chacun un nombre entier de 0 à 5 à la condition que l'un au moins des p et q est 0 ; et à condition que, dans le composé de formule (III), p et q ne puissent pas être 0 en même temps, quand R est l'hydrogène ; 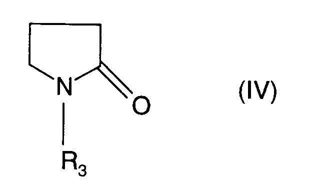
où R₃ représente un groupe alkyle à chaîne linéaire ou ramifiée ayant de 8 à 18 atomes de carbone.

2. Une composition pour le traitement des cheveux selon la revendication 1, selon laquelle ledit composant (a) est en quantité de 0,01 à 50 % en poids et ledit composé (b) est en quantité de 0,1 à 50 % en poids.

3. Une composition pour le traitement des cheveux selon la revendication 1, selon laquelle ledit composant (a) est un mélange d'un composé de formule générale (I) et d'un composé de formule générale (II) et un rapport pondéral entre le composé de formule générale (I) au composé de formule générale (II) (le composé de formule générale (I)/le composé de formule générale (II)) est dans l'intervalle de 1/10 à 10/1.

4. Une composition pour le traitement des cheveux selon la revendication 1, selon laquelle un rapport pondéral entre ledit composant (a) et ledit composant (b), ((a)/(b)), est dans l'intervalle de 1/20 à 20/1.

5. Une composition pour le traitement des cheveux selon l'une quelconque des revendications 1 à 4, selon laquelle additionnellement un composant (c) contenu, étant représenté par 1 ou plusieurs composés choisis dans le groupe comprenant l'ammoniac, les composés amino, les amines et les sels d'ammonium quaternaire ; et selon laquelle la valeur du pH est ajustée à 8 à 11 au moment de l'emploi.

6. Une composition pour le traitement des cheveux selon la revendication 5, selon laquelle ladite composition pour le traitement des cheveux est fournie sous la forme d'une composition du type à deux emballages constitués de :
un premier emballage comprenant ledit composant (a), ou ledit composant (a) et ledit composant (b) ; et
un second emballage comprenant ledit composant (b) et le composant (c), ou ledit composant (c).

7. Une composition pour le traitement des cheveux selon la revendication 5, selon laquelle ledit composant (c) est en quantité de 0,1 à 10 % en poids.

8. Une composition pour le traitement des cheveux qui comprend :
(a) un ou plusieurs composés représentés par la formule générale (I) ou (II) suivante ; et
(b) un ou plusieurs composés représentés par la formule générale suivante (III) ou (IV) ; et
(d) un ou plusieurs agents oxydants : 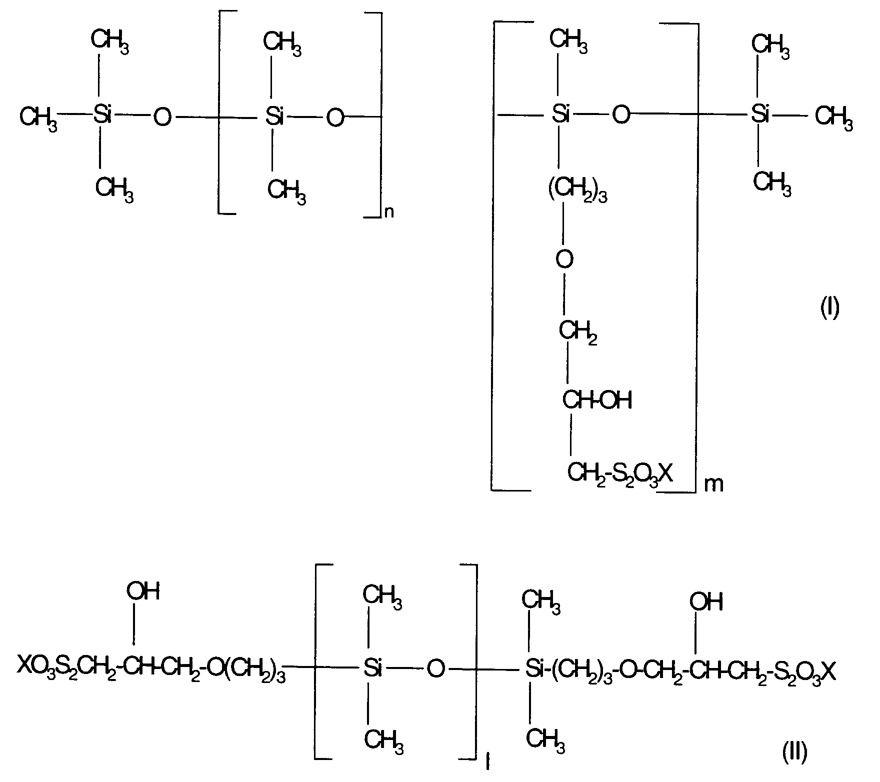
dans laquelle X représente un métal alcalin, un métal alcalino-terreux, un ammonium, un ammonium primaire, un ammonium secondaire, un ammonium tertiaire ou un ammonium quaternaire ; n est un nombre entier de 1 à 20 ; m est un nombre entier de 1 à 20, à la condition que n/m>1 ; et l est un nombre entier de 1 à 20 ; 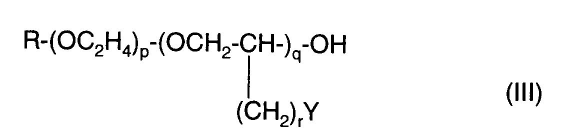
où R représente un atome d'hydrogène ou un groupe 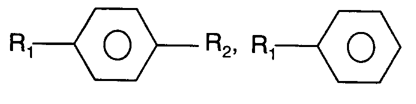 ou un groupe alkyle à chaîne linéaire ou ramifiée ayant de 1 à 4 atomes de carbone, dans lequel R₁ représente un atome d'hydrogène, un groupe méthyle ou un groupe méthoxy, et R₂ représente un groupe -CH₂-, -C(CH₃)₂-, -CH₂-CH(CH₃)- ou -CH=CH-CH₂- ; Y représente un atome d'hydrogène ou un groupe hydroxyle ; et p, q et r désignent chacun un nombre entier de 0 à 5 à la condition que l'un au moins des p et q est 0 ; et à condition que, dans le composé de formule (III), p et q ne puissent pas être 0 en même temps, quand R est l'hydrogène ; 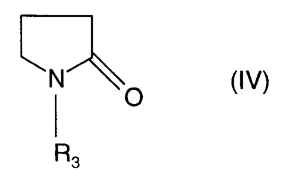
où R₃ représente un groupe alkyle à chaîne linéaire ou ramifiée ayant de 8 à 18 atomes de carbone.

9. Une composition pour le traitement des cheveux selon la revendication 8, selon laquelle ladite composition pour le traitement des cheveux est présentée sous la forme d'une composition du type à deux emballages constitués de :
un premier emballage comprenant ledit composant (a), ou ledit composant (a) et ledit composant (b) ;
un second emballage comprenant ledit composant (b) et ledit composant (d), ou ledit composant (d).

10. Une composition pour le traitement des cheveux selon la revendication 8, selon laquelle ledit composant (a) est en quantité de 0,01 à 50 % en poids, ledit composant (b) est en quantité de 0,1 à 50 % en poids et ledit composant (d) est en quantité de 0,05 à 30 % en poids.

11. Une composition pour le traitement des cheveux selon la revendication 8, selon laquelle ledit composant (a) est un mélange d'un composé de formule générale (I) et d'un composé de formule générale (II) et un rapport pondéral entre le composé de formule générale (I) et le composé de formule générale (II) (le composé de formule générale (I)/le composé de formule générale (II)) est dans l'intervalle de 1/10 à 10/1.

12. Une composition pour le traitement des cheveux selon la revendication 8, selon laquelle un rapport pondéral entre ledit comp osant (a) et ledit composant (b) ((a)/(b)) est dans l'intervalle de 1/20 à 20/1.

13. Une silicone cyclique modifiée au sel de Bunte représentée par la formule générale suivante (V) : 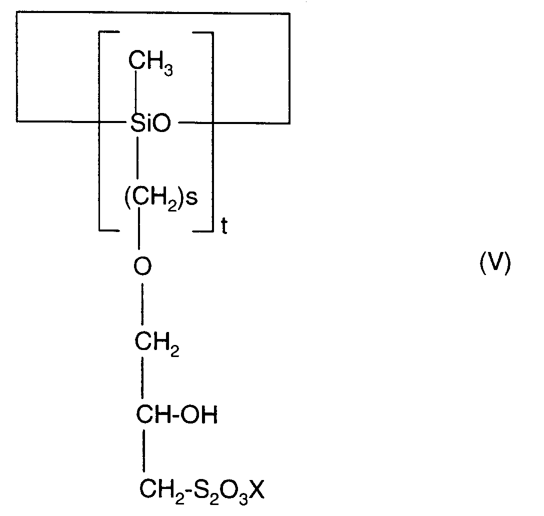
dans laquelle X représente un métal alcalin, un métal alcalino-terreux, un ammonium, un ammonium primaire, un ammonium secondaire, un ammonium tertiaire ou un ammonium quaternaire ; t est un nombre entier de 4 à 10 ; et s est un nombre entier de 3 à 11.

14. Une composition pour le traitement des cheveux qui comprend une ou plusieurs silicones cycliques modifiées au sel de Bunte telles que définies dans la revendication 13, et dont la valeur de pH est ajustée à 2 à 4 ou à 8 à 10 au moment de l'emploi.

15. Une composition pour le traitement des cheveux selon la revendication 14, selon laquelle une ou plusieurs silicones cycliques modifiées au sel de Bunte sont en quantité de 0,01 à 50 % en poids.

16. Une composition pour le traitement des cheveux qui comprend :
(e) une ou plusieurs silicones cycliques modifiées au sel de Bunte telles que définies dans la revendication 13 ; et
(d) un ou plusieurs agents oxydants.

17. Une composition pour le traitement des cheveux selon la revendication 16, selon laquelle ladite composition pour le traitement des cheveux est présentée sous la forme d'une composition du type à deux emballages constitués de :
un premier emballage comprenant ledit composant (e) ; et
un second emballage comprenant ledit composant (d).

18. Une composition pour le traitement des cheveux selon la revendication 16, selon laquelle ledit composant (e) est en quantité de 0,01 à 50 % en poids et ledit composant (d) est en quantité de 0,05 à 30 % en poids.

19. Une composition pour le traitement des cheveux qui comprend :
(e) une ou plusieurs silicones cycliques modifiées au sel de Bunte telles que définies dans la revendication 13 ; et
(f) un ou plusieurs agents réducteurs.

20. Une composition pour le traitement des cheveux selon la revendication 19, selon laquelle ladite composition pour le traitement des cheveux est présentée sous la forme d'une composition du type a deux emballages constitués de :
un premier emballage comprenant ledit composant (e) ; et
un second emballage comprenant ledit composant (f).

21. Une composition pour le traitement des cheveux selon la revendication 19, selon laquelle ledit composant (e) est en quantité de 0,01 à 50 % en poids et ledit composant (f) est en quantité de 0,1 à 20 % en poids.
